# FASCICULE DE BREVET EUROPEEN

(11) **EP 0 873 309 B1**
(45) Date de publication et mention de la délivrance du brevet: **18.12.2002**
(21) Numéro de dépôt: 96934967.9
(22) Date de dépôt: 24.10.1996
(51) Int. Cl.: C07D 209/96, A61K 31/40

(54) **DERIVES 3-SPIRO-INDOLIN-2-ONE COMME LIGANDS DES RECEPTEURS DE LA VASOPRESSINE ET/OU DE L'OCYTOCINE**
3-SPIRO-INDOLIN-2-ONDERIVATE ALS LIGANDEN DER VASOPRESSIN UND/ODER DER OCYTOCIN REZEPTOREN
3-SPIRO-INDOLIN-2-ONE DERIVATIVES AS VASOPRESSIN AND/OR OXYTOCIN RECEPTOR LIGANDS

(30) Priorité: 24.10.1995 FR 9512533
(43) Date de publication de la demande: 28.10.1998
(73) Titulaire: SANOFI-SYNTHELABO, 75013 Paris (FR)
(72) Inventeur: FOULON, Loic, F-31120 Pinsaguel (FR); GARCIA, Georges, F-34110 Frontignan (FR); SERRADEIL-LE GAL, Claudine, F-31750 Escalquens (FR); VALETTE, Gérard, F-31120 Lacroix-Falgarde (FR)
(74) Mandataire: Gillard, Marie-Louise
(86) Numéro de dépôt international: FR9601666
(87) Numéro de publication internationale: WO97015556

(56) Documents cités:
- EP-A- 0 636 608
- EP-A- 0 636 609
- WO-A-93/15051

## Description

La présente invention a pour objet de nouveaux dérivés d'indolin-2-one, un procédé pour leur préparation. Ces nouveaux dérivés sont pourvus d'affinité pour les récepteurs de la vasopressine et/ou de l'ocytocine et peuvent donc constituer des principes actifs de compositions pharmaceutiques.

La vasopressine est une hormone connue pour son effet antidiurétique et son effet dans la régulation de la pression artérielle. Elle stimule plusieurs types de récepteurs : V₁ (V₁ₐ, V_{1b} ou V₃), V₂. Ces récepteurs sont localisés dans le foie, les vaisseaux (coronaires, rénaux, cérébraux), les plaquettes, le rein, l'utérus, les glandes surrénales, le système nerveux central, l'hypophyse. L'ocytocine a une structure peptidique proche de celle de la vasopressine. Les récepteurs de l'ocytocine se trouvent aussi sur le muscle lisse de l'utérus ; ils se trouvent également sur les cellules myoépithéliales de la glande mammaire, dans le système nerveux central et dans le rein. La localisation des différents récepteurs est décrite dans : Jard S. et al., "Vasopressin and oxytocin receptors : an overview in Progress" dans Endocrinology., Imura H. and Shizume K. ed., Experta Medica, Amsterdam, 1988, 1183-1188, ainsi que dans les articles suivants : Presse Médicale, 1987, *16* (10), 481-485, J. Lab. Clin. Med., 1989, *114* (6), 617-632 et Pharmacol. Rev., 1991 *43* (1), 73-108. La vasopressine exerce ainsi des effets hormonaux, cardiovasculaires, hépatiques, rénaux, antidiurétiques, agrégants et des effets sur les systèmes nerveux central et périphérique, sur les sphères utérine et intestinale et sur le système oculaire et pulmonaire. L'ocytocine intervient dans la parturition, la lactation et le comportement sexuel.

Les antagonistes des récepteurs V₂ de la vasopressine (appelés également "AVP-2-antagonistes" ou "antagonistes V₂") sont préconisables comme puissants aquarétiques qui interviennent spécifiquement sur la réabsorption rénale de l'eau sans entrainer de fuites électrolytiques (Na⁺, K⁺) comme le font les diurétiques classiquement utilisés en clinique, tels que le furosemide ou l'hydrochlorothiazide. Ces derniers entrainent après un traitement prolongé des hypokaliémies et hyponatrémies.

Le premier antagoniste des récepteurs V₂ de l'arginine-vasopressine (ci-après dénommée AVP) : l'OPC-31260, est actuellement en cours de développement clinique. La comparaison des effets de l'OPC-31260 aux diurétiques classiques, tel que le furosemide, démontre que, tant chez l'animal (Yoshitaka Y. et al., Br. J. Pharmacol., 1992, *105*, 787-791) que chez l'homme (Akihiro O. et al., J. Clin. Invest., 1993, 92, 2653-2659, Akihiro O. et al., J. Pharmacol. Exp. Ther., 1995, 272, 546-551) un tel composé favorise sélectivement la diurèse aqueuse et n'affecte pas, ou très peu aux fortes doses, l'excrétion des ions.

Des dérivés d'indolin-2-one ont été décrits dans la littérature. A titre d'exemple, on peut citer le brevet ZA 830952 qui décrit des dérivés utiles comme antihypertenseurs qui inhibent l'enzyme de conversion, le brevet FR 1509373 qui décrit des composés diurétiques pourvus d'un effet sur l'excrétion du potassium.

Plusieurs demandes de brevet ou brevets décrivent également des séries de composés non peptidiques possédant une affinité pour les récepteurs de la vasopressine et/ou de l'ocytocine. C'est le cas par exemple de EP 382185 qui décrit des dérivés de carbostyryle qui sont des antagonistes de la vasopressine utiles comme vasodilatateurs, hypotenseurs, diurétiques et antiagrégants plaquettaires ; de EP 444945 qui décrit des dérivés de spiropipéridine utiles notamment dans la dysménorrhée ; de EP 514667 qui décrit des dérivés de benzazépine utiles notamment dans les troubles de la fonction rénale, dans l'hyponatrémie, le diabète ou encore dans le traitement et la prophylaxie de l'hypertension et dans l'inhibition de l'agrégation plaquettaire ; de JP 03127732 qui décrit des dérivés d'indoles comme antagonistes de la vasopressine.

Des dérivés de benzyle ou sulfonylindoline et d'indole ont également été décrits comme antagonistes de la vasopressine. A cet effet, on peut citer les documents EP 469984, EP 526348, EP 636608, EP 636609, WO 93/15051 et WO 95/18105, mais ces documents ne décrivent pas des composés actifs de façon sélective sur le récepteur AVP-2.

Il a été maintenant trouvé que certaines indolinones présentent une excellente affinité vis-à-vis des récepteurs de la vasopressine et/ou de l'ocytocine. Ces nouvelles indolin-2-ones sont des AVP-2-antagonistes puissants et sélectifs. De plus, compte-tenu de leur structure et en particulier de la présence de diverses fonctions polaires, notamment des fonctions salifiables, ces molécules possèdent une bonne dispersibilité et/ou solubilité dans l'eau qui leur confère une activité pharmacologique améliorée et permettent aussi la préparation aisée de formes galéniques injectables.

Ainsi, selon l'un de ses aspects, la présente invention concerne de nouvelles indolin-2-ones répondant à la formule : 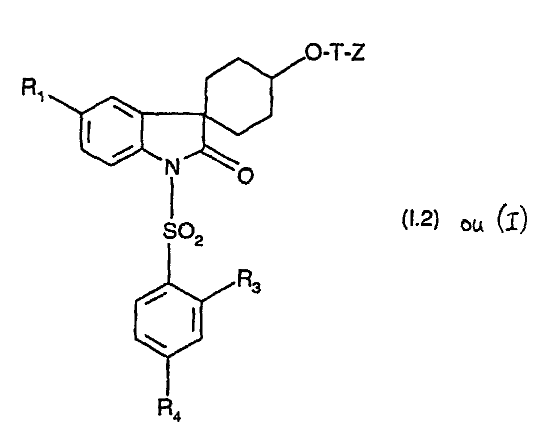 dans laquelle :
- R₁ représente un hydrogène ; un hydroxyle ; un halogène ; un (C₁-C₇)alkyle ; un (C₁-C₇)polyfluoroalkyle ; un (C₁-C₇)alcoxy ; un (C₁-C₇)alkylthio ; un (C₁-C₇)polyfluoroalcoxy ; un (C₃-C₇)cycloalkyloxy ; un (C₃-C₇)cycloalkylthio ; un cycloalkylméthoxy ou un cycloalkylméthylthio dans lesquels le cycloalkyle est en C₃-C₇ ; un phénoxy ; un benzyloxy ; un nitro ; un cyano ;
- R₃ et R₄ représentent chacun indépendamment l'un de l'autre un hydrogène ; un halogène ; un (C₁-C₇)alkyle ; un (C₂-C₇)alcényle ; un (C₁-C₇)polyhalogénoalkyle ; un phényle ou un benzyle ; un cyano ; un nitro ; un groupe -NR₅R₆ ; un hydroxyamino ; un hydroxyle ; un groupe OR₇ ; un groupe SR₇ ; un groupe -COOR₈ ; un groupe -CONR₉R₁₀ ; un groupe -CSNR₉R₁₀ ; l'un au moins des radicaux R₃ et R₄ étant différent de l'hydrogène ;
- R₅ et R₆ représentent chacun indépendamment un hydrogène ; un (C₁-C₇)alkyle ; un (C₂-C₇)alcényle ; un phényle ; un benzyle ; un (C₁-C₇)alkylcarbonyle ; un (C₁-C₇)alkylthiocarbonyle ; un (C₃-C₇)cycloalkylcarbonyle ; un (C₃-C₇)cycloalkylthiocarbonyle ; un benzoyle ; un thiénylcarbonyle ; un furylcarbonyle ; un (C₁-C₇)alkyloxycarbonyle ; un phénoxycarbonyle ; un benzyloxycarbonyle ; un carbamoyle ou un thiocarbamoyle non substitué ou substitué par R₉ et R₁₀; ou bien R₅ et R₆ constituent avec l'atome d'azote auquel ils sont liés un groupe hétérocyclique choisi parmi les groupes pyrrolidine, pyrroline, pyrrolyle, indoline, indole, pipéridine ;
- R₇ représente un (C₁-C₇)alkyle ; un (C₂-C₇)alcényle ; un phényle ; un benzyle ; un (C₃-C₇)cycloalkyle ; un (C₁-C₇)polyfluoroalkyle ; un formyle ; un (C₁-C₇)alkylcarbonyle ; un benzoyle ; un benzylcarbonyle;
- R₈ représente un hydrogène, un (C₁-C₇)alkyle ; un phényle ; un benzyle ;
- R₉ et R₁₀ représentent chacun indépendamment l'hydrogène ; un (C₁-C₇)alkyle ; un (C₁-C₇)polyfluoroalkyle ; un (C₂-C₇)alcényle ; un (C₃-C₇)cycloalkyle éventuellement substitué par un groupe hydroxy (C₁-C₄)alkyle ; un pyridyle ; un phényle ; un thiényle ; un furyle ; un (C₄-C₇)azacycloalkyle ; ou bien R₉ et R₁₀ constituent avec l'atome d'azote auquel ils sont liés un groupe hétérocyclique choisi parmi les groupes pyrrolidine, pipéridine et pipérazine, non substitués ou substitués par des (C₁-C₄)alkyles ;
- T représente un (C₁-C₄)alkylène éventuellement interrompu par un (C₃-C₆)cycloalkylène, lesdits alkylènes étant éventuellement substitués une ou plusieurs fois sur le même atome de carbone par un (C₁-C₃)alkyle ; ou bien T représente une liaison directe ;
- Z représente un groupe -NR₁₁R₁₂ ; -⁺NR₁₁R₁₂(C₁-C₄)alkyle (A⁻), (A⁻) étant un anion, de préférence Cl⁻, Br⁻, I⁻ ou CH₃SO₄⁻ ; -N(O)R₁₁R₁₂ ; un groupe -COOR₁₁ ; un groupe -NR₁₁COR₁₂ ; un groupe -CONR₁₁R₁₂, étant entendu que lorsque T représente un méthylène ou une liaison directe, Z ne peut pas être -NR₁₁R₁₂ ; -⁺NR₁₁R₁₂(C₁-C₄)alkyle (A⁻); -N(O)R₁₁R₁₂ ; NR₁₁COR₁₂;
- R₁₁ et R₁₂ représentent chacun indépendamment l'hydrogène ; un (C₁-C₇)alkyle ; un (C₁-C₄)alcoxy ; un (C₃-C₇)cycloalkyle ; un phényle ; un (C₁-C₃)alkylène-cycloalkyle dans lequel le cycloalkyle est en C₃-C₇; un (C₁-C₃)alkylènephényle; lesdits groupes pouvant éventuellement être mono- ou poly-substitués par R₁₃;
ou bien R₁₁ et R₁₂ constituent éventuellement avec l'atome d'azote auquel ils sont liés un hétérocycle choisi parmi les hétérocycles azétidine, pyrrolidine, pipéridine, pipérazine, pipérazinone, morpholine, morpholinone, thiomorpholine, hexahydroazépine, éventuellement mono-substitué par R₁₃ ;
- R₁₃ représente un (C₁-C₄)alkyle ; un hydroxyle ; un hydroxyalkyloxy ; un (C₁-C₄)alcoxy ; un groupe -NR₁₄R₁₅ dans lequel R₁₄ et R₁₅ représentent chacun indépendamment l'hydrogène ou un (C₁-C₄)alkyle ; un amidino ; un guanidino ; un imidazolyle ; un pyridyle ; un indolyle ; un tétrahydroisoquinolyle ;
- le groupe phényle constitutif des substituants R₁, R₃, R₄, R₅, R₆, R₇, R₈, R₉, R₁₀, R₁₁ et R₁₂ étant non substitué, mono- ou di-substitué par un (C₁-C₇)alkyle, un (C₁-C₇)alkoxy, un trifluorométhyle, un halogène ou trisubstitué par un (C₁-C₇)alkyle, un (C₁-C₇)alkoxy ou un halogène;
ainsi que leurs sels, solvates ou hydrates.

On notera que les composés dans lesquels
―O―T―Z est 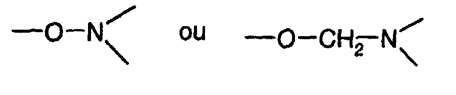 sont instables et ne font donc pas partie de l'invention.

Selon la présente invention, par "(C₁-C₇)alkyle" ou "(C₁-C₆)alkyle", on entend un alkyle droit ou ramifié ayant 1 à 7 atomes de carbone ou respectivement 1 à 6 atomes de carbone.

Selon la présente invention, par halogène on entend un atome choisi parmi le fluor, le chlore, le brome ou l'iode, de préférence le fluor ou le chlore.

Lorsqu'un composé selon l'invention présente un ou des carbones asymétriques, les isomères optiques de ce composé font partie intégrante de l'invention.

Lorsqu'un composé selon l'invention présente une stéréoisomérie par exemple de type axial-équatorial ou Z-E, l'invention comprend tous les stéréoisomères de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate.

Les sels des composés de formule (I) comprennent également des sels avec des bases organiques ou minérales, par exemple les sels des métaux alcalins ou alcalinoterreux, comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Les groupes fonctionnels éventuellement présents dans la molécule des composés de formule (I) et dans les intermédiaires réactionnels peuvent être protégés, soit sous forme permanente soit sous forme temporaire, par des groupes protecteurs qui assurent une synthèse univoque des composés attendus.

Par groupe protecteur temporaire des aminés, alcools, phénols, thiols ou des acides carboxyliques on entend les groupes protecteurs tels que ceux décrits dans Protective Groups in Organic Synthesis, Greene T.W. et Wuts P.G.M., ed. John Wiley et Sons. 1991 et dans Protective Groups, Kocienski P.J., 1994. Georg Thieme Verlag.

On peut citer par exemple des groupements protecteurs temporaires des amines benzyles, carbamates (tels que *tert*-butyloxycarbonyl clivables en milieu acide, benzyloxycarbonyle, clivables par hydrogénolyse), des acides carboxyliques (esters d'alkyle tels méthyle ou éthyle, *tert*-butyle hydrolysables en milieu basiques ou acides, benzyliques hydrogènolysables), des alcools ou des phénols (tels que des éthers de tétrahydropyranyle ou méthyloxyméthyle ou méthyléthoxyméthyle, *tert*-butyl et benzyle) et se référer aux méthodes générales bien connues décrites dans Protective Groups, cité ci-dessus.

On préférera selon la présente invention les groupements protecteurs temporaires clivables en milieu acide ou en milieu neutre et par hydrogénolyse.

Les groupes protecteurs permanents sont ceux qui sont stables dans les conditions de clivage cités ci-dessus et qui sont susceptibles d'être présents dans les produits finaux. De tels groupes O-protecteurs ou N-protecteurs sont constitués par les groupes (C₁-C₇)alkyle, phényle. Les groupes N-protecteurs permanents incluant également les groupes (C₁-C₅)alcanoyles et les groupes aroyles, tel que le benzoyle.

Les composés (I) peuvent comporter des groupes précurseurs d'autres fonctions qui sont générées ultérieurement en une ou plusieurs autres étapes.

Les composés de formule (I) dans lesquels les diverses fonctions polaires, notamment les fonctions salifiables qui améliorent la solubilité et/ou la disponibilité dans l'eau sont de préférence portés par le groupe -T-Z.

Les composés de formule (I) préférés sont les composés de formule (I) dans lesquels R₁ représente un atome de chlore ou un groupe éthoxy.

Les composés de formule (I) dans lesquels R₃ représente l'hydrogène ou un méthoxy et R₄ représente un groupe méthoxy, diéthyluréido, *tert*-amylcarbamoyle et *tert*-butylcarbamoyle sont des composés préférés.

Tout particulièrement préférés sont les composés de formule : 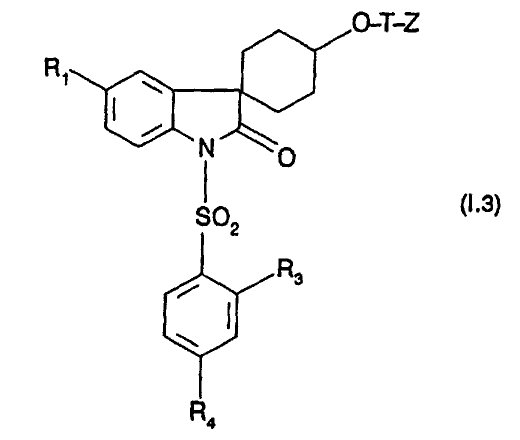 dans laquelle R₁, R₃ et R₄ sont tels que définis pour (I), T représente un (C₁-C₃)alkylène et Z représente un groupe amino, un 2-hydroxyéthylamino, un 2-(2-hydroxy)éthyloxyéthylamino, un morpholinyle ou un groupe carboxylique, et leurs sels, solvates ou hydrates.

Plus particulièrement préférés sont les composés de formule : 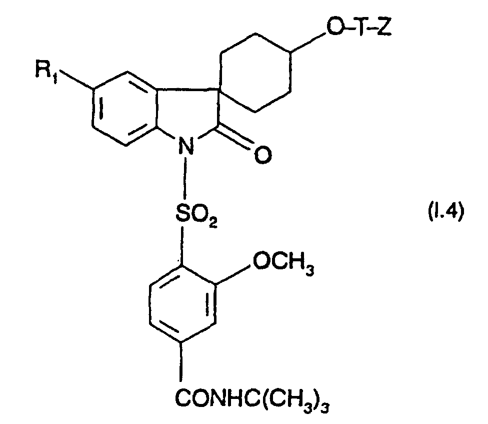 dans laquelle R₁, T et Z sont tels que définis pour (I) et leurs sels, solvates et hydrates.

Les composés de formules (I.1), (I.2), (I.3) et (I.4) dans lesquelles :
- R₁ représente un atome de chlore ou un groupe éthoxy ;
- T représente un (C₁-C₃)alkylène et Z représente un groupe amino, un 2-hydroxyéthylamino, un 2-(2-hydroxy)éthyloxyéthylamino, un morpholinyle ou un groupe carboxylique, sont particulièrement préférés.

Sont également préférés les composés de formule (I.2) et (I.3) dans lesquelles :
- R₁ représente un atome de chlore ou un groupe éthoxy ;
- R₃ représente l'hydrogène ou un méthoxy ;
- R₄ représente un groupe méthoxy, diéthyluréido, *tert*-amylcarbamoyle, et *tert*-butylcarbamoyle.

Parmi ces composés, on préfère ceux dans lesquels T représente un (C₁-C₃)alkylène et Z représente un groupe amino, un 2-hydroxyéthylamino, un 2-(2-hydroxy)éthyloxyéthylamino, un morpholinyle ou un groupe carboxylique.

Tout particulièrement préférés sont les composés ci-après :
* 5-Chloro-3-spiro-[4-(2-morpholinoéthyloxy)cyclohexane]-1-[4-(N-*tert*-butylcarbamoyl)-2-méthoxybenzènesulfonyl]indolin-2-one ;
* 5-Ethoxy-3-spiro-[4-(2-aminoéthyloxy)cyclohexane]-1-[4-(N-*tert*-butylcarbamoyl)-2-méthoxybenzènesulfonyl]indolin-2-one ;
* 5-Ethoxy-3-spiro-[4-(2-(N-méthyl-N-(2-hydroxyéthyl)amino) éthyloxy)cyclohexane]-1-[4-(N-*tert*-butylcarbamoyl)-2-méthoxybenzène sulfonyl]indolin-2-one ;
* 5-Ethoxy-1-[4-(N-*tert*-butylcarbamoyl)-2-méthoxybenzènesulfonyl]-3-spiro-[4-(2-morpholinoéthyloxy)cyclohexane]indolin-2-one;
* 5-Ethoxy-3-spiro-(4-carboxyméthyloxycyclohexane)-1-(4-N-*tert*-butylcarbamoyl-2-méthoxybenzènesulfonyl)indolin-2-one ;
* 5-Ethoxy-3-spiro-[4-(2-morpholinoéthyloxy)cyclohexane]-1-[4-(N-*tert*-amylcarbamoyl)-2-méthoxybenzènesulfonyl]indolin-2-one;
* 5-Ethoxy-3-spiro-[4-(2-carboxyéthyloxy)cyclohexane]-1-[4-(N*-tert*-amylcarbamoyl)-2-méthoxybenzènesulfonyl]indolin-2-one ;
* 5-Ethoxy-1-[4-(N',N'-diéthyluréido)-2-méthoxybenzènesulfonyl]-3-spiro-[4-(2-diméthylaminoéthyloxy)cyclohexane]indolin-2-one ;
* 5-Ethoxy-3-spiro-[4-(2-(4-éthoxypipéridino)éthyloxy)cyclohexane]-1-[4-(N-*tert*-butylcarbamoyl)-2-méthoxybenzènesulfonyl]indolin-2-one;
* 5-Ethoxy-3-spiro-[4-(2-glycylaminoéthyloxy)cyclohexane]-1-[4-(N-*tert*-butylcarbamoyl)-2-méthoxybenzènesulfonyl]indolin-2-one ;
* 5-Ethoxy-3-spiro-[4-(2-(N,N-diméthylglycylamino)éthyloxy)cyclohexane]-1-[4-(N-*tert*-butylcarbamoyl)-2-méthoxybenzènesulfonyl]indolin-2-one ;
* 5-Chloro-3-spiro-[4-(N-(3-diméthylaminopropyl)carbamoylméthyloxy)cyclohexane]-1-[4-(N-*tert*-butylcarbamoyl)-2-méthoxybenzènesulfonyl]indolin-2-one ;
* 5-Ethoxy-3-spiro-[4-(2-(4-diméthylaminobutyrylamino)éthyloxy)cyclohexane]-1-[4-(N-*tert*-butylcarbamoyl)-2-méthoxybenzènesulfonyl]indolin-2-one ;
* 5-Ethoxy-3-spiro-[4-(2-(2-hydroxyéthylamino)éthyloxy)cyclohexane]-1-[4-(N-*tert*-butylcarbamoyl)-2-méthoxybenzènesulfonyl]indolin-2-one;
* 5-Ethoxy-3-spiro-[4-(2-(2-(2-hydroxyéthyloxy)éthylamino)éthyloxy) cyclohexane]-1-[4-(N-*tert*-butylcarbamoyl)2-méthoxybenzènesulfonyl]indolin-2-one ;
ainsi que leurs sels, solvates ou hydrates pharmaceutiquement acceptables étant particulièrement adaptés pour l'utilisation dans des formulations pharmaceutiques.

Les composés selon l'invention peuvent être préparés selon le SCHEMA 1 ci-après, dans lequel :
R₁, R₃, R₄, T et Z sont tels que définis pour (I) on (I.2);
R₂ est un atome d'hydrogène;
W est SO₂;
Cy constitue avec le carbone auquel il est lié un cyclohexane substitué en position 4 par rapport au carbone spiro par -O-T-Z, -O-T-X, -OH, etc.

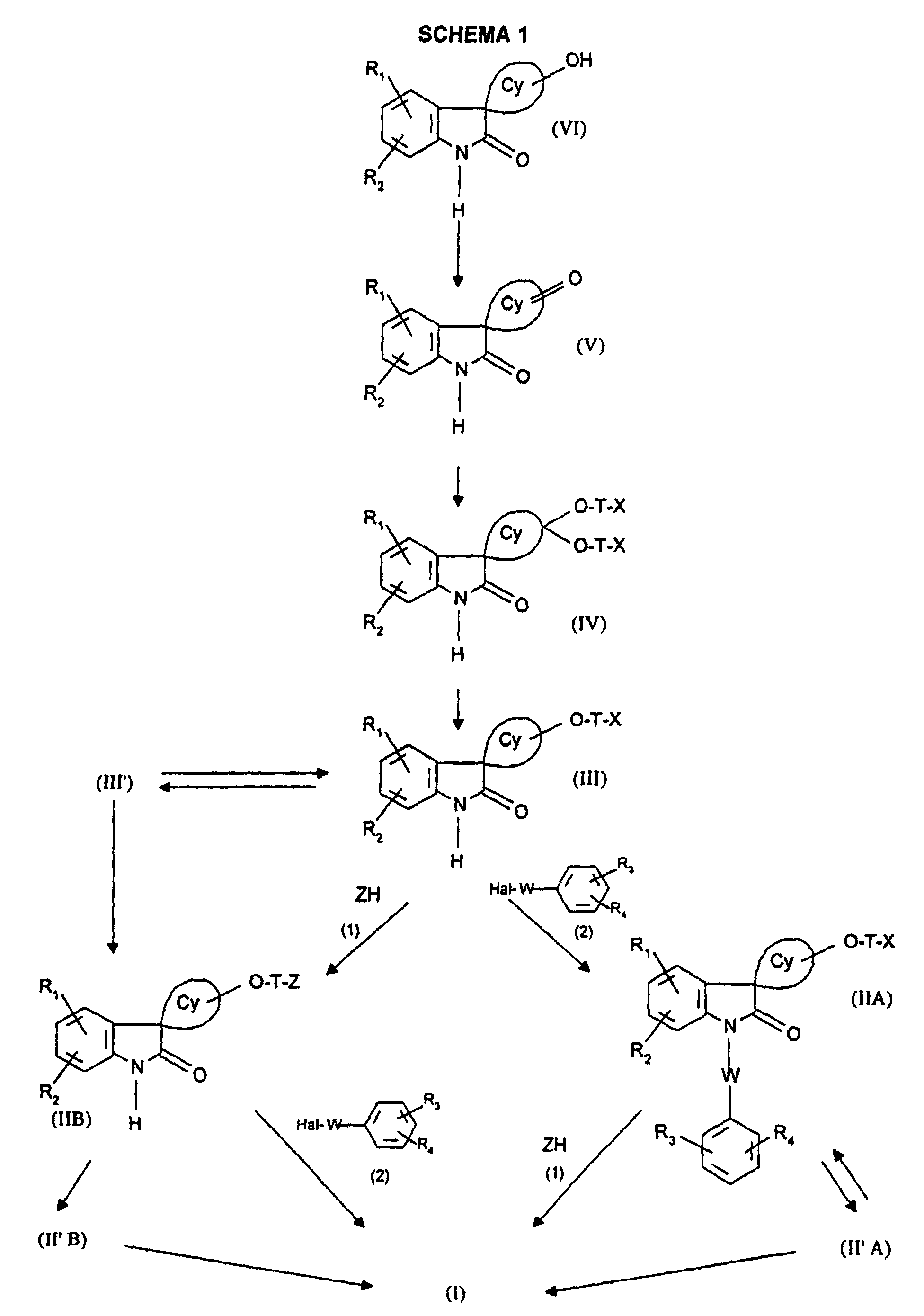

La présente invention a également pour objet un procédé pour la préparation des composés de formule (I) selon l'invention, caractérisé en ce que :
(1) soit on fait réagir sur un composé de formule : 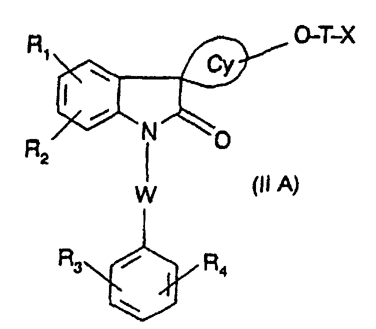 dans lequel R₁, R₂, R₃, R₄, W, Cy et T sont tels que définis précédemment et dans lequel X est un halogène, de préférence brome, chlore ou iode, ou un tosyloxy, mésyloxy avec un dérivé de formule ZH (1) dans lequel Z est NR₁₁R₁₂ tel que défini pour (I) et au moins l'un des radicaux R₁₁ et R₁₂ est différent de l'hydrogène, dans le diméthylformamide, le tétrahydrofurane ou l'acétonitrile, à des températures comprises entre 0° et 120°C, ou bien X représente un azido que l'on réduit en amino ;
(2) soit lorsque Z = -COOH on fait réagir un composé de formule : 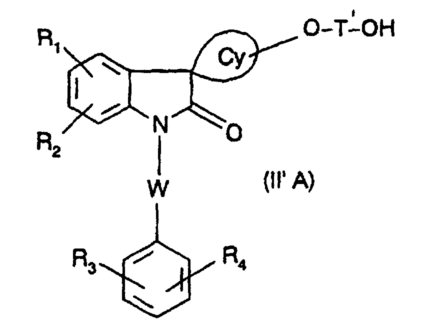 dans laquelle R₁, R₂, W, R₃, R₄, et Cy sont tels que définis précédemment et T' représente T-CH₂- avec un oxydant, tel que l'oxyde de chrome dans un solvant acide, tel que l'acide acétique dilué à une température comprise entre 0° et 100°C, les bichromates alcalins ou les permanganates alcalins ou alcalino terreux ;
(3) soit on fait réagir un composé de formule : 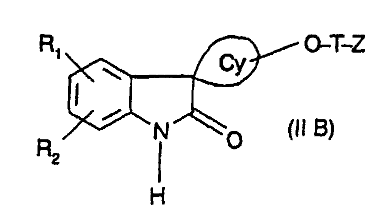 dans lequel R₁, R₂, Cy, T et Z sont tels que définis précédemment avec un composé de formule: 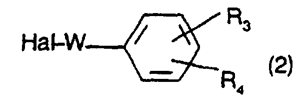 dans lequel W, R₃ et R₄ sont tels que définis précédemment et Hal représente un atome d'halogène en présence d'un hydrure métallique comme par exemple l'hydrure de sodium ou d'un alcoolate alcalin comme par exemple le *tert-*butylate de potassium à des températures comprises entre -40° et 25°C, dans un solvant anhydre tel que le tétrahydrofurane ;
(4) soit lorsque Z = -COOH on fait réagir un composé de formule: 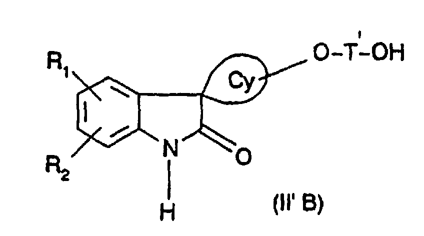 dans laquelle R₁, R₂, et Cy sont tels que définis précédemment et T' représente T-CH₂ avec un oxydant décrit ci-dessus pour la transformation de (II' A) en (I) puis ensuite, on protège éventuellement l'acide ainsi obtenu de formule : 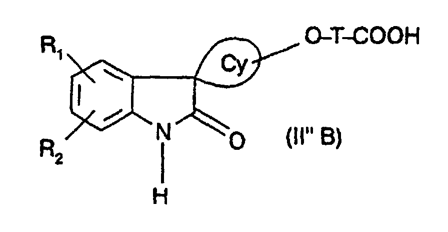 dans lequel R₁, R₂, Cy et T sont tels que définis précédemment par un groupement protecteur de l'acide carboxylique pour obtenir l'intermédiaire de formule : 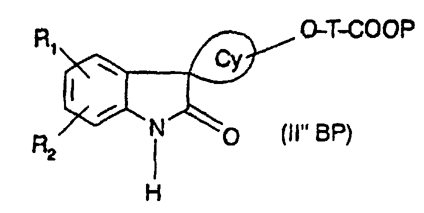 dans lequel R₁, R₂, Cy et T sont tels que définis précédemment et P représente un groupe protecteur choisi parmi un alkyle, tel qu'un *tert*-butyle ou un benzyle et on soumet enfin ce composé (II"BP) à l'action d'un dérivé de formule (2), pour obtenir après déprotection un composé (I) ; qui est éventuellement transformé en l'un de ses sels selon les techniques bien connues de l'homme du métier.

Les composés (II A) et (II B) peuvent être préparés à partir des composés (III) selon le SCHEMA 2 suivant :

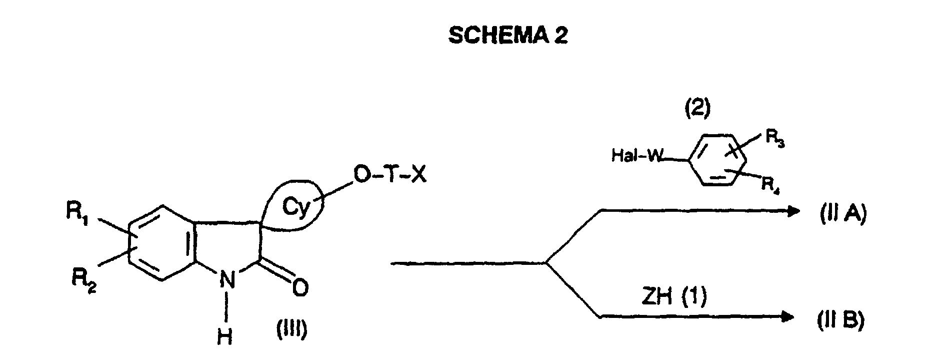

Les composés (II A) peuvent être préparés à partir de l'indolin-2-one (III) avec un halogénure de benzènesulfonyle dans un solvant anhydre, tel que le diméthylformamide ou le tétrahydrofurane en présence d'un hydrure métallique, tel que l'hydrure de sodium ou d'un alcoolate alcalin comme par exemple le *tert*-butylate de potassium à des températures comprises entre -40° et 25°C.

Les composés (II A) peuvent être également préparés à partir des alcools (II' A) selon des méthodes générales connues. On peut citer par exemple des systèmes triphénylphosphine / tétrachlorure de carbone selon Angew. Chem. Int. Ed., 1975, *14,* 801 ou triphénylphosphine / C(Hal)₄ dans lequel Hal représente un halogène en présence de pyridine selon Carbohyd. Res., 1978, *61*, 511 ou par réaction avec un halogénure d'aryle- ou d'alkyl-sulfonyle en présence d'une base dans un solvant inerte. Les groupes X peuvent s'échanger : par exemple on peut transformer un groupe sulfonate en un halogénure, tel qu'un dérivé de l'iode par une réaction avec un iodure alcalin tel que l'iodure de sodium selon J. Chem. Soc., 1949, 326. Lorsque X représente un halogène on peut transformer l'halogénure (II A) en alcool (II' A) par substitution par un ion nitrate qui est ensuite réduit en présence d'un catalyseur métallique, tel que le palladium sur charbon selon la méthode décrite dans J. Med. Chem., 1995, *38*, 130-136.

On peut également préparer les composés de formule (II' A) à partir des indolin-2-ones (III') correspondantes par réaction avec les réactifs (2) dans les conditions déjà décrites pour la transformation des composés (III) en (II A). La fonction alcool de (III') sera temporairement protégée (composés III' P), par exemple par un groupe protecteur, tel que méthyle ou tétrahydropyranyle selon EP 636 608.

Les composés (II B) peuvent être préparés à partir de l'indolin-2-one (III) par substitution du groupe nucléofuge X par un dérivé ZH(1) tel que par exemple une amine primaire ou secondaire, dans des solvants polaires, tels que le diméthylformamide, le tétrahydrofurane ou l'acétonitrile, à des températures comprises entre 0° et 120°C en fonction de la nature du nucléophile et du nucléofuge.

Les composés (II B) pour lesquels -TZ représente -T-COOH sont préparés à partir d'un alcool (III') dans lequel T' représente T-CH₂- en oxydant l'alcool (III') selon les conditions décrites pour la transformation de (II'A) en (I).

Les composés (III), sont originaux et font partie de l'invention. Ils peuvent être préparés selon le SCHEMA 3 réactionnel ci-après :

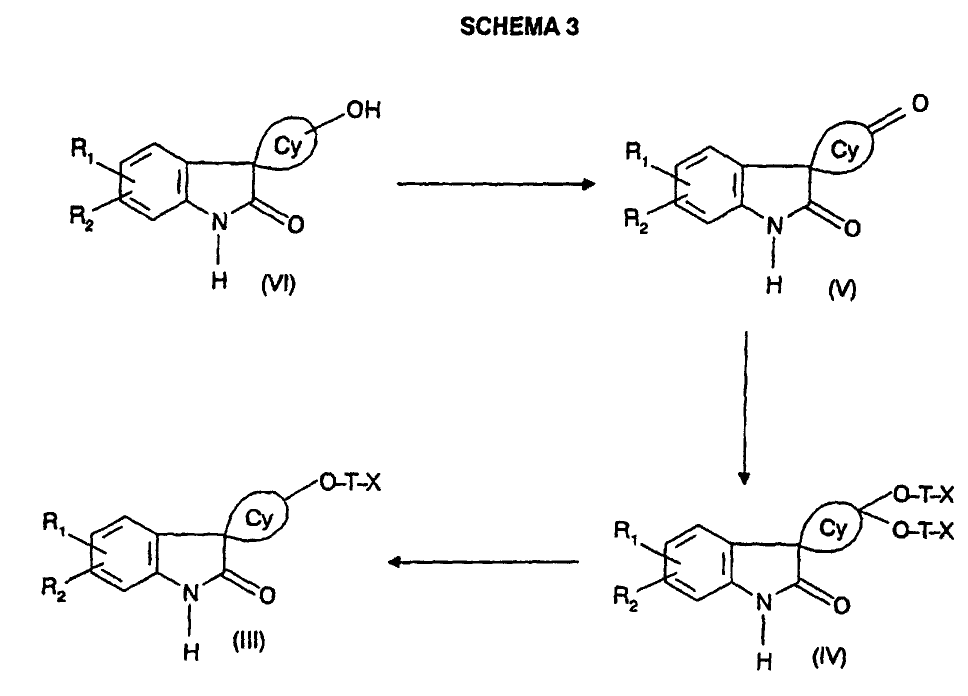

Ainsi, les indolin-2-ones (III) peuvent être obtenues par réduction des acétals (IV) dans des conditions douces par exemple selon la méthode décrite dans J. Org. Chem., 1987, *52*, 2594-2596 par l'action du borohydrure de zinc en présence de chlorure de triméthylsilane dans des éthers ou des solvants chlorés, tels que par exemple le dichlorométhane, ou par l'action du complexe diméthylsulfure. BH₃ en présence de triflate de triméthylsilyle dans les éthers ou le dichlorométhane selon la méthode décrite dans J. Org. Chem., 1993, *58,* 6756-6765, ou à partir des alcools (III'): 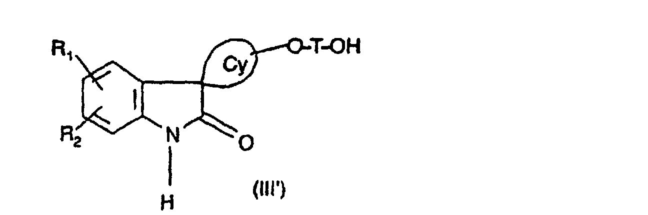 dans laquelle R₁, R₂, Cy et T sont tels que définis précédemment selon les méthodes citées précédemment pour la transformation de (II' A) en (II A).

Les acétals (IV) sont préparés par des réactions bien connues par exemple à partir d'une cétone (V) sur un alcool par catalyse acide en milieu déshydratant. On peut opérer par élimination azéotropique d'eau ou en présence de tamis moléculaires selon Synthesis 1972, 419.

Les cétones (V) peuvent être préparées à partir des alcools secondaires correspondants (VI) selon les nombreuses méthodes bien connues de l'homme de l'art mettant par exemple en jeu des oxydants tels que l'oxyde de chrome en milieu acétique ou des complexes de l'oxyde de chrome tels que le chlorochromate de pyridinium dans des solvants inertes tels que l'acétate d'éthyle ou le dichlorométhane ou bien encore par hydrolyse des acétals (IV').

Les alcools (VI) peuvent être obtenus à partir des composés correspondants dont la fonction hydroxy est protégée, par exemple par un groupe méthoxyméthyle ou tétrahydropyranyle. Ces composés sont décrits dans EP 636608 ou obtenus de manière similaire. On soumet les composés ainsi protégés de formule : 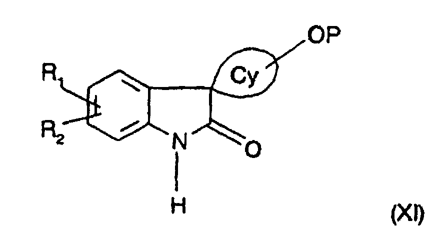 à une hydrolyse chlorhydrique dans un alcool, tel que le méthanol ou l'éthanol ou dans un éther tel que le tétrahydrofurane à des températures comprises entre -5° et 70°C.

Les composés (III') peuvent être préparés selon le SCHEMA 4 ci-après :

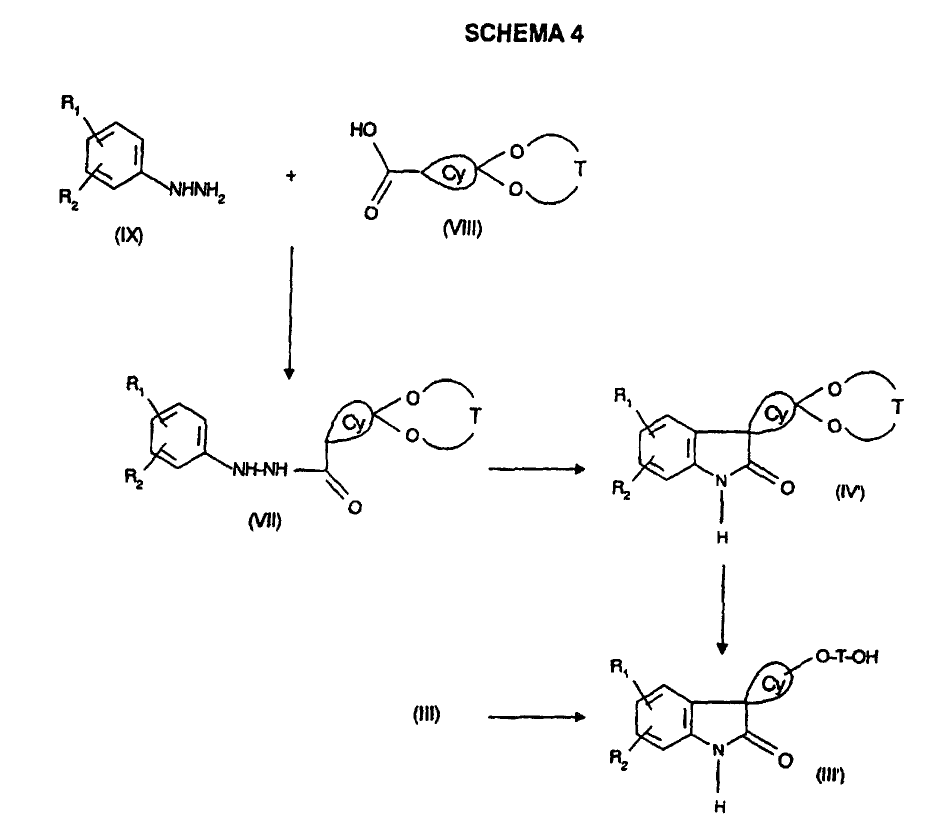

Comme pour la préparation des composés (III) à partir des acétals (IV) on peut préparer les composés (III') à partir d'un acétal cyclique (IV') tel qu'un dioxolane qui est obtenu à partir d'un hydrazide (VII).

On peut également transformer un halogénure (III) en (III') selon les méthodes déjà citées pour la transformation des composés (II A) en composés (II'A).

A l'inverse, et comme pour les méthodes déjà citées pour la transformation des composés (II'A) en (II A) on peut également transformer les alcools (III') en composés (III) dans lesquels X est un groupe nucléofuge tel que alkyle ou benzènesulfonate par réaction avec un halogénure d'alkyle ou de phénylsulfonyle dans des solvants inertes en présence d'une amine tertiaire ou dans la pyridine.

Les composés (III') peuvent être transformés en composés (III' P) dont la fonction alcool est protégée comme indiqué précédemment. Les composés (III'P) peuvent également avec les réactions décrites précédemment être transformés en composés (II A) dans lesquels X est un alcool protégé temporairement.

Les composés (IV') dans lesquels T est au moins égal à -CH₂CH₂-peuvent être préparés à partir des cétones (V) par réaction avec un diol HO-T-OH selon les conditions citées pour la transformation de (V) en (IV). Les composés (IV') peuvent également être obtenus directement à partir des hydrazides correspondants (VII) par une réaction de Brunner décrite par Moore R.F. et al., J. Chem. Soc., 1951, 3475-3478, par exemple par chauffage dans des solvants tels que la quinoléine en présence d'un oxyde métallique ou alcalino-terreux comme l'oxyde de calcium. On peut également procéder par chauffage dans des solvants inertes tels que la tétraline, le naphtalène ou le 1,2,3,4-tétraméthylbenzène selon la méthode décrite par Wolff J. et al., Tetrahedron, 1986, *42*, (15), 4267-4272, à partir d'un sel de lithium préparé au préalable dans un solvant inerte tel que le tétrahydrofurane à basse température.

Ces dérivés de phénylhydrazide (VII) peuvent être obtenus à partir d'une phénylhydrazine (IX), qui sont des composés connus ou préparés selon des méthodes connues, et de dérivés des acides carboxyliques (VIII), tels que les esters, chlorures ou anhydrides mixtes obtenus par réaction d'un chloroformiate d'alkyle, de préférence isobutyle, en présence d'une base selon les méthodes classiques bien connues de l'homme de l'art. Les acides (VIII) sont connus ou préparés selon des méthodes connues.

Une alternative pour la synthèse des composés (I) dans lesquels T représente -CH₂- et Z représente un groupe -COOZ₁ dans lequel Z₁ représente l'hydrogène, un (C₁-C₃)alkyle ou un benzyle, consiste à utiliser un alcool de formule : 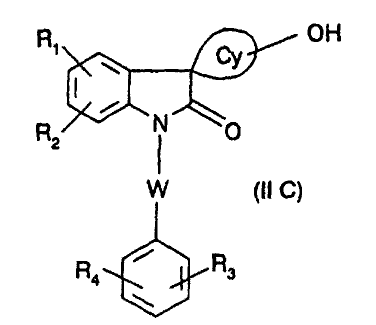 dans laquelle R₁, R₂, R₃, R₄, W et Cy sont tels que définis précédemment, qui sont des produits connus ou préparés selon EP 636609, sur lesquels on fait une alkylation avec un alkylant puissant tel qu'un trifluorométhanesulfonate de formule CF₃SO₂O-CH₂-COO Alk (3) généré *in situ* par réaction du triflate d'argent sur le dérivé halogéné correspondant dans lequel Alk représente un (C₁-C₄) alkyle, dans des solvants halogénés tels que dichlorométhane ou le tétrachlorure de carbone en présence d'une base telle que la 2,6-di-*tert*-butylpyridine selon la méthode décrite pour les trifluorométhanesulfonates d'alkyles dans Carbohydrate Research, 1975, *44*, C5-C7.

L'ester ainsi obtenu peut être échangé ou clivé dans les conditions générales déjà citées.

Les alcools (II C) peuvent être préparés selon le SCHEMA 5 suivant :

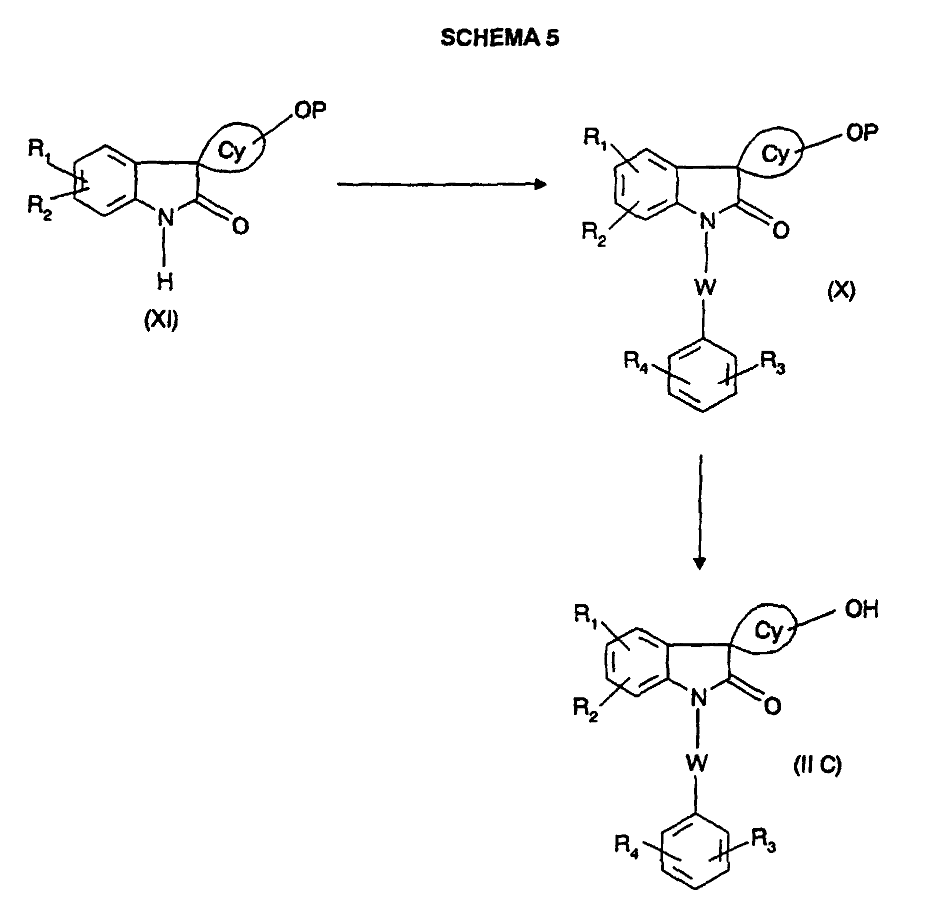

Les alcools (II C) peuvent être préparés à partir des composés protégés (X) par déprotection dans les mêmes conditions que pour la transformation des composés (XI) en composés (VI).

Les composés (X) sont obtenus à partir des composés (XI) selon le procédé décrit dans EP 636608 avec les halogénures (2) selon les conditions déjà décrites pour la transformation des composés (II B) en (I) et des composés (III) en (II A).

On peut également transformer un composé de formule (I) en un autre composé de formule (I) portant un résidu polytonctionnel tel que défini pour Z, en particulier pour -NR₁₁COR₁₂ ou pour -CONR₁₁R₁₂ en procédant selon les méthodes connues de la synthèse peptidique décrite par exemple par Bodansky M., dans Principles of Peptide Synthesis 2^{nd} ed., 1993 ; et Bodansky M., dans Peptide Chemistry dans Springer Verlag ; ainsi ces méthodes permettent d'éviter la racémfsation des centres asymétriques portés éventuellement par les aminoacides.

Les réactifs ZH de formule (1) sont commerciaux ou préparés selon des méthodes connues.

Les dérivés de formule (2) : 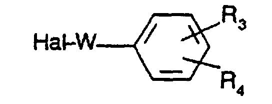 sont également préparés selon des méthodes connues. Notamment, les halogénures de benzènesulfonyle dans lesquels W = -SO₂- et R₃ et R₄ sont tels que définis précédemment pour (I) sont préparés par des méthodes connues. Ainsi par exemple, le chlorure de 4-diméthylaminobenzènesulfonyle est préparé selon Sukenik C.N. et al., J. Am. Chem. Soc., 1977, 99, 851-858. Plus généralement, les halogénures de benzènesulfonyle substitués par un groupe diméthylamino sont connus ou préparés par des méthodes connues ; le chlorure de 4-benzyloxybenzènesulfonyle est préparé selon EP 229 566.

Le chlorure d'alcoxybenzènesulfonyle est préparé à partir de l'alcoxybenzènesulfonate de sodium, lui-même préparé par action d'un halogénure d'alkyle sur l'hydroxybenzènesulfonate de sodium.

On obtient les halogénures de benzènesulfonyle selon Col. Czechoslov. Chem. Commun., 1984, *49*, 1184, à partir des dérivés de l'aniline substitués par le même groupement, lesdits dérivés de l'aniline étant eux-mêmes obtenus à partir des dérivés nitrés correspondants.

L'halogénure de benzènesulfonyle (2) dans lequel le substituant en position 4 représente un groupe -NHCON(CH₂CH₃)₂ peut être préparé par action de l'acide chlorosulfonique sur la N',N'-diéthyl-N-phénylurée, elle-même obtenue par réaction de l'aniline avec le chlorure de diéthylcarbamoyle.

Dans le cas où R₃ ou R₄ représentent un carbamoyle N-substitué, on peut condenser un composé (2) dans lequel R₃ est un précurseur d'acide carboxylique, tel que N-benzylcarbamoyle, déprotèger le groupement protecteur par hydrogénolyse puis condenser avec l'amine désirée ou bien préparer directement (2) dans lequel R₃ à la valeur attendue. On opère généralement à partir des anilines correctement choisies, elles mêmes étant obtenues par réduction des dérivés nitrés correspondants.

Les anilines sont diazotées dans les conditions classiques par l'acide nitreux et mises en réaction avec du SO₂ en présence de chlorure cuivrique selon J. Heterocyclic Chem., 1986, *23,* 1253.

On obtient les composés (3) à partir d'un iodoacétate d'alkyle et d'un sel de l'acide trifluorométhane sulfonique tel que le sel d'argent selon Chem. Reviews, 1977, 77.

Les ammoniums quaternaires, les dérivés N-oxydes, S-oxydes et les sulfones des composés (I) font partie de l'invention et sont préparés classiquement respectivement par réaction avec un halogénure d'alkyle, par oxydation avec de l'eau oxygénée ou un peracide, tel que l'acide peracétique ou métachloroperbenzoïque dans des solvants inertes.

Les composés de formule (I) peuvent comporter des fonctions amines ou acides qui peuvent être transformées en fonctions amides par réactions avec respectivement des dérivés d'acides ou d'amides pouvant comporter des carbones asymétriques. On se référera alors aux réactions de couplage non racémisantes bien connues de l'homme de l'art notamment dans la synthèse peptidique et on consultera Wunsch E., dans Methoden der Organischen Chemie (Synthèse von Peptiden), 1974, 15, band 1+2, Thieme Verlag, Stuttgart ou Jones J.H., dans The Peptides, 1979, *1*, 65-104, Gross E., Meienhofer J., Academic Press, ou M. Bodansky, Principles of Peptide Synthesis et Peptide Chemistry, 1993, Springer Verlag.

Les composés de formule (I) ci-dessus comprennent également ceux dans lesquels un ou plusieurs atomes d'hydrogène, de carbone ou d'halogène, notamment de chlore ou de fluor ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligands de récepteurs.

L'affinité des composés selon l'invention pour les récepteurs V₁ de la vasopressine a été déterminée *in vitro* en utilisant la méthode décrite dans Lynch C.J. et al., J. Biol. Chem., 1985, *260* (5), 2844-2851. Cette méthode consiste à étudier le déplacement de la vasopressine tritiée fixée aux sites V₁ de membranes de foie de rat.

De même, l'affinité des composés (I) selon l'invention pour les récepteurs de l'ocytocine a été déterminée *in vitro* par déplacement d'un analogue radioiodé de l'ocytocine fixé aux récepteurs d'une préparation membranaire de glandes mammaires de rates en gestation, selon une technique proche de celle décrite par Elands J. *et al.* dans Eur. J. Pharmacol., 1987, *147*, 197-207.

L'affinité des composés (I) selon l'invention pour les récepteurs V₂ a été mesurée sur une préparation membranaire de rein de boeuf selon une méthode adaptée de Crause P. et al., Molecular and Cellular Endocrinology, 1982, *28*, 529-541 et de Stassen F.L. et al., J. Pharmacol. Exp. Ther., 1982, *233*, 50-54.

Les composés selon l'invention inhibent la fixation de l'arginine-vasopressine tritiée, aux récepteurs de la préparation membranaire. Les Cl₅₀ des composés selon l'invention sont faibles, allant généralement de 10⁻⁵ à 10⁻⁹ M.

L'activité agoniste ou antagoniste des récepteurs de la vasopressine des composés selon l'invention, administrés par voie orale, a été évaluée chez le rat normalement hydraté (souche Sprague-Dawley) selon la technique décrite dans Br. J. Pharmacol., 1992, *105*, 787-791. L'effet diurétique, observé en général pour les composés de formule (I) et, pour certains de ces composés, à des doses inférieures ou égales à 10 mg/kg, montre que les composés de formule (I) constituent une série de puissants antagonistes V₂.

Les composés selon l'invention sont actifs après administration par différentes voies, notamment par voie orale.

Aucun signe de toxicité n'est observé avec ces composés aux doses pharmacologiquement actives et leur toxicité est donc compatible avec leur utilisation médicale comme médicaments.

Les composés selon la présente invention permettent, soit de mimer, soit d'inhiber, de façon sélective, les effets de la vasopressine et/ou de l'ocytocine. Parmi ces composés les antagonistes des récepteurs de la vasopressine peuvent intervenir sur la régulation de la circulation centrale et périphérique, notamment les circulations coronaire, rénale et gastrique, ainsi que sur la régulation hydrique et la libération de l'hormone adrénocorticotrophique (ACTH). Les agonistes de la vasopressine peuvent remplacer avantageusement la vasopressine ou ses analogues dans le traitement du diabète insipide ; ils peuvent également être utilisés dans le traitement de l'énurésie, et dans la régulation de l'hémostase : traitement de l'hémophilie, du syndrome de Von Willebrand, antidote des agrégants plaquettaires, Laszlo F.A., Pharmacol. Rev., 1991, *43,* 73-108. Drug Investigation, 1990, *2* (suppl. 5), 1-47. Les hormones elles-mêmes : la vasopressine et l'ocytocine ainsi que certains de leurs analogues peptidiques ou non peptidiques sont utilisés en thérapeutique et ont montré leur efficacité (Vasopressin. Gross P. et al. ed. John Libbey Eurotext, 1993, en particulier 243-257 et 549-562. Laszlo F.A. and Laszlo F.A. Jr., Clinical perspectives for vasopressin antagonists, Drug News Perspect., 1993, 6 (8) ; North W.G., J. Clin. Endocrinol., 1991, *73*, 1316-1320. Legros J.J. et al., Prog. Neuro-Pharmacol. Biol. Psychiat., 1988, *12,* 571-586 ; Andersson K.E. et al., Drugs Today, 1988, *24* (7), 509-528 ; Stump D.L. et al., Drugs, 1990, *39*, 38-53 ; Caltabiano S. et al., Drugs Future, 1988, *13*, 25-30 ; Mura Y. et al., Clin. Nephrol. 1993, *40*, 60-61 ; Faseb J., 1994, *8* (5), A587 : 3398).

Ce type de molécules antagonistes V₂ à profil aquarétique possède un large éventail d'indications thérapeutiques et constitue une innovation majeure dans les traitements de l'insuffisance cardiaque, des hyponatrémies, des désordres hydriques, des rétentions d'eau, etc. Ce type de composé peut remplacer avantageusement les diurétiques classiques dans toutes les pathologies où ils sont préconisés chez l'homme et chez l'animal. On peut aussi envisager avec de telles molécules le traitement de l'hypertension en association avec des anti-hypertenseurs d'autres classes thérapeutiques comme par exemple des β-bloquants, des inhibiteurs de l'enzyme de conversion ou encore des antagonistes des récepteurs de l'angiotensine II.

Ainsi les composés selon l'invention sont utiles notamment dans le traitement des affections des systèmes nerveux central et périphérique, du système cardiovasculaire, du système endocrinien et hépatique, de la sphère rénale, de la sphère gastrique et intestinale et pulmonaire, en ophtalmologie et dans les troubles du comportement sexuel, chez l'homme et chez l'animal.

La présente invention a donc également pour objet des compositions pharmaceutiques contenant une dose efficace d'un composé selon l'invention ou d'un sel, d'un solvate ou d'un hydrate pharmaceutiquement acceptable de celui-ci, et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique, rectale ou intraocculaire, les principes actifs de formule (I) ci-dessus, ou leurs sels, solvates et hydrates éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec des matrices telles qu'un polymère ou une cyclodextrine (patch, formes à libération prolongée).

Les compositions selon l'invention peuvent être utilisés dans le traitement ou la prévention de différentes affections vasopressine-dépendantes ou ocytocine-dépendantes ainsi que dans les dysfonctionnements de la sécrétion de la vasopressine ou d'ocytocine, les affections cardiovasculaires, comme l'hypertension, l'hypertension pulmonaire, l'insuffisance cardiaque, l'insuffisance circulatoire, l'infarctus du myocarde, l'athérosclérose ou le vasospasme coronaire, en particulier chez le fumeur, les angines instables et PTCA (percutaneous transluminal coronary angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, la boulimie, les états psychotiques, les troubles de la mémoire par exemple ; les rénopathies et les dysfonctionnements rénaux comme les oedèmes, le vasospasme rénal, la nécrose du cortex rénal, le syndrome néphrotique, les hyponatriémies, l'hypokaliémie, le diabète, le syndrome de Schwartz-Bartter ou la lithiase rénale ; les affections du système gastrique, comme le vasospasme gastrique, l'hypertension portale, l'hépatocirrhose, les ulcères, la pathologie des vomissements, par exemple la nausée y compris la nausée due à une chimiothérapie, le mal des transports, ou encore le syndrome de la sécrétion inappropriée de l'hormone antidiurétique (SIADH), le diabète insipide et l'énurésie ; les affections du système hépatique tel que les cirrhoses du foie ; les ascites abdominales et tous les désordres provoquant une rétention d'eau anormale ; les désordres surrénaliens (maladie de Cushing) et en particulier l'hypercorticisme et l'hyperaldostéronémie. Les compositions selon l'invention peuvent également être utilisés dans le traitement des troubles du comportement sexuel, dans la surcharge pondérale ou l'excès de poids et l'obésité en remplaçant avantageusement les diurétiques classiques déjà utilisés pour cette indication. Chez la femme, les compositions selon l'invention peuvent être utilisées pour traiter la dysménorrhée ou le travail prématuré. On peut également utiliser les compositions selon l'invention dans le traitement des cancers pulmonaires à petites cellules, des encéphalopathies hyponatriémiques, de la maladie de Raynaud, du syndrome de Menière, du syndrome pulmonaire, du glaucome et de la prévention de la cataracte et dans les traitements post-opératoires, notamment après une chirurgie abdominale, cardiaque ou hémorragique.

Les compositions de la présente invention peuvent contenir, à côté des produits de formule (I) ci-dessus ou de leurs sels, solvates et hydrates pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur le système rénine-angiotensine tel qu'un inhibiteur de l'enzyme de conversion, un antagoniste de l'angiotensine II, un inhibiteur de la rénine. On peut également associer un composé selon l'invention, par exemple, avec un vasodilatateur périphérique, un inhibiteur calcique, un béta-bloquant, un alpha-1-bloquant ou diurétique. De telles compositions seront utiles en particulier dans le traitement de l'hypertension ou de la défaillance cardiaque. On peut également associer deux composés selon l'invention : un antagoniste spécifique du récepteur V₁ à un antagoniste spécifique de l'ocytocine ou un antagoniste V₁ et un antagoniste V₂ ou un antagoniste V₂ et un agoniste V₁.

De façon avantageuse les compositions de la présente invention contiennent un produit de formule (I.3) ou (I.4) ci-dessus ou un de ses sels, solvates ou hydrates pharmaceutiquement acceptable. Chacun de ces composés peut également être associé à un antagoniste spécifique de l'angiotensine Il de préférence à l'irbésartan.

Ces associations permettront de renforcer les activités thérapeutiques des composés selon l'invention.

Les PREPARATIONS et EXEMPLES suivants illustrent l'invention sans toutefois la limiter.

Les spectres de résonance magnétique nucléaire ont été effectués dans le DMSO-d6 sauf mention contraire, à 200 MHz et les déplacements chimiques sont exprimés en p.p.m.

Les abréviations utilisées ci-après sont les suivantes :
s = singulet
m = multiplet
t = triplet
q = quintuplet

### PREPARATION 1 Alcools de formule (VI)

### 5-Ethoxy-3-spiro-(4-hydroxycyclohexane)indolin-2-one. Composé (VI.1)

On chauffe à 40°C durant 3 heures une solution de 22 g de 5-éthoxy-3-spiro-(4-méthoxyméthyloxycyclohexane)indolin-2-one préparé selon EP 636608 dans 130 ml de méthanol et 9 ml d'acide chlorhydrique concentré (36 %). On refroidit le mélange réactionnel, puis successivement on essore, rince à l'éther diéthylique et sèche le précipité pour obtenir l'isomère polaire du produit attendu ; F = 225°C. On ajoute 50 ml d'eau au filtrat, puis successivement on évapore le méthanol, extrait au dichlorométhane, lave les phases organiques à l'eau, sèche et évapore pour obtenir le produit attendu sous forme d'un mélange d'isomères ; F = 170°C.

### 5-Chloro-3-spiro-(4-hydroxycyclohexane)indolin-2-one. Composé (VI.2)

On procède selon le même mode opératoire que précédemment à partir de la 5-chloro-3-spiro-(4-méthoxyméthyloxycyclohexane)indolin-2-one préparé à partir de 5-chloroindolin-2-one selon la méthode décrite dans EP 636608. On isole après extraction au dichlorométhane le produit attendu sous forme d'un mélange d'isomères ; F = 260°C

### PREPARATION 2 Cétones de formule (V)

### 5-Ethoxy-3-spiro-(4-oxocyclohexane)indolin-2-one. Composé (V. 1)

On dissout 3,8 g de 5-éthoxy-3-spiro-(4-hydroxycyclohexane)indolin-2-one *(VI.1)* (mélange d'isomères) et 5,8 ml de pyridine dans 250 ml d'acétate d'éthyle et on ajoute 6,3 g de chlorochromate de pyridinium adsorbé sur 29 g d'alumine neutre. On agite ensuite le mélange réactionnel à 25°C durant 16 heures, puis on filtre et on évapore le solvant du filtrat. On isole 3,4 g du produit attendu après recristallisation en présence de charbon actif dans le toluène ; F = 168°C.

### 5-Chloro-3-spiro-(4-oxocyclohexane)indolin-2-one. Composé (V.2)

On prépare ce composé selon le même mode opératoire que pour la préparation du *composé (V.1)* à partir de 5-chloro-3-spiro-(4-hydroxy)cyclohexane) indolin-2-one *(VI.2)* ; F = 220°C.

### PREPARATION 3 Acétals de formule (IV)

### 5-Ethoxy-3-spiro-[4,4-di-(2-chloroéthyloxy)cyclohexane]indolin-2-one. Composé (IV.1)

On solubilise 3 g de 5-éthoxy-3-spiro-(4-oxocyclohexane)indolin-2-one *(V.1)* dans 30 ml de toluène et on ajoute 4,6 ml de 2-chloroéthanol, 20 g de tamis moléculaire 5 Å et 0,22 g d'acide méthane sulfonique. On agite le mélange réactionnel lentement durant 18 heures à 20°C, puis on filtre et on rince le tamis moléculaire avec du dichlorométhane. On évapore le solvant puis cristallise le produit attendu dans l'éther diéthylique ; F = 170°C.

### 5-Ethoxy-3-spire-[4,4-di(3-chloropropyloxy)cyclohexane]indolin-2-one. Composé (IV.2)

On procède selon le même mode opératoire que pour la préparation du *composé (IV.1)* à partir de la même cétone *(V.1)* et de 3-chloropropanol ; F = 147°C.

### 5-Chloro-3-spiro-[4,4-di-(2-chloroéthyloxy)cyclohexane]indolin-2-one. Composé (IV.3)

On procède selon le même mode opératoire que pour la préparation du *composé (IV.1)* à partir du *composé (V.2)* et de 2-chloroéthanol ; F = 174°C.

### PREPARATION 4 Dérivés de formule (III)

### 5-Ethoxy-3-spiro-[4-(3-chloropropyloxy)cyclohexane]indolin-2-one (mélange d'isomères). Composé (III.1)

A 0°C, on ajoute lentement 2,2 ml d'une solution de borohydrure de zinc 0.29 M dans l'éther diéthylique (préparée selon la méthode décrite dans Chem. Pharm. Bull. 1984, *32* (4), 1411-1415) à 0,55 g d'acétal *IV.2* dans 3 ml de dichlorométhane puis 0,34 ml de triméthylchlorosilane. On agite le mélange réactionnel pendant 16 heures à 20°C, puis successivement on ajoute 10 ml d'une solution saturée de NaHCO₃, on extrait à l'acétate d'éthyle et on lave les phases organiques avec une solution saturée de NaCI. Après séchage sur MgSO₄ et évaporation on isole 0,4 g d'une huile qui est chromatographiée sur gel de silice en éluant avec un mélange cyclohexane/acétate d'éthyle 8/2 (v/v). On isole le produit attendu (mélange d'isomères) sous forme de résine
¹H RMN, CDCl₃, 200 MHz : 7,75 (s,1H) ; 7,03 (d, 0,25H) ; 6,83 (d, 0,75H) ; 6,79-6,65 (m, 3H) ; 4,06-3,9 (q, 2H) ; 3,72-3,58 (m, 4H) ; 3,54-3,50 (m, 1H) ; 2,18-1,53 (m, 10H) ; 1,37 (t, 3H).

### 5-Ethoxy-3-spiro-[4-(2-chloroéthyloxy)cyclohexane]indolin-2-one (mélange d'isomères). Composé (III.2)

On procède selon le même mode opératoire que pour la préparation du *composé (III.1)* à partir du *composé (IV.1)*.
¹H RMN, CDCl₃, 200 MHz : 8 (s, 1H); 6,85-6,63 (m, 3H) ; 4,03-3,93 (q, 2H) ; 3,81-3,74 (m, 2H) ; 3,70-3,58 (m, 3H) ; 2,21-1,55 (m, 8H) ; 1,4 (t, 3H).

### 5-Chloro-3-spiro-[4-(2-chloroéthyloxy)cyclohexane]indolin-2-one. (mélange d'isomères) Composé (III.3)

On procède selon le même mode opératoire que pour la préparation du *composé* (*III.1)* à partir du *composé* (*IV.3).*
¹H RMN, DMSO-d6 200 MHz : 10,49 (s, 0,25H) ; 10,39 (s, 0,75H) ; 7,40 (s, 1H); 7,21-7,16 (d, 1H) ; 6,81-6,77 (d, 1H) ; 3,7 (m, 4H) ; 3,55 (m, 1H) ; 1,96-1,61 (m, 8H).

### 5-Ethoxy-3-spire-[4-(2-tosyloxyethyloxy)cyclohexane]indolin-2-one. Composé (III.4)

On ajoute à 0°C, 17,97 g de chlorure de tosyle à 19,25 g de *(III'1)* décrit dans la PREPARATION 10 dans 130 ml de pyridine. On agite à 20°C pendant 3 heures. On coule le mélange réactionnel sur 650 ml d'eau puis on agite pendant 30 minutes. On isole 28,06 g du produit attendu après filtration, lavages à l'eau et séchage à 40°C sous vide en présence d'anhydride phosphorique. Le produit obtenu à partir de l'isomère polaire *(III'1)* fond à 152°C.

### PREPARATION 5 Dérivés de formule (II A)

### 5-Ethoxy-1-[4-(N-tert-butylcarbamoyl))-2-méthoxybenzènesulfonyl))-3-spiro-[4-(2-chloroéthyloxy)cyclohexane]indolin-2-one (mélange d'isomères). Composé (II A. 1)

On ajoute 0,29 g de *tert*-butylate de potassium à une solution refroidie à -60°C de 0,75 g de dérivé chloré *(III.2)* et 0,75 g de chlorure de 4-(N-*tert*-butylcarbamoyl)-2-méthoxybenzènesulfonyle dans 90 ml de tétrahydrofurane. On laisse remonter la température à 20°C, on agite le mélange réactionnel pendant 2 heures, puis on ajoute 30 ml d'une solution à 15% de NaCI et successivement on extrait à l'acétate d'éthyle, lave les phases organiques avec une solution à 15% de NaCI, sèche les phases organiques sur MgSO₄, évapore le solvant et chromatographie le résidu sur gel de silice en éluant avec un mélange cyclohexane/acétate d'éthyle 85/15 (v/v) pour isoler le produit attendu sous forme de résine.
¹H RMN, DMSO-d6 200 MHz : 8 (m, 2H) ; 7,5 (m, 3H) ; 7,04 (s, 0,75H) ; 6,85 (m, 1,25H) ; 4,0 (q, 2H) ; 3,6 (s, 3H) ; 3,66 (s, 4H) ; 3,58 ( s, 3H) ; 3.5 (m, 1H) ; 1,9-1,6 (m, 8H) ; 1,34 (s, 9H) ; 1,28 (t, 3H).

### 5-Ethoxy-1-[4-(N',N'-diéthyluréido)-2-méthoxybenzènesulfonyl]-3-spiro-[4-(2-tosyloxyéthyloxy)cyclohexane] indolin-2-one. Composé (II A.2)

On ajoute à 0°C, 0,25 g de chlorure de tosyle à une solution de 0,18 ml de triéthylamine et 0,25 g de 5-éthoxy-1-[4-(N',N'-diéthyluréido)-2-méthoxy benzènesulfonyl]-3-spiro-[4-(2-hydroxyéthyloxy)cyclohexane]indolin-2-one (préparé dans EP 0636608) dans 3 ml de tétrahydrofurane anhydre. On agite le mélange réactionnel durant 48 heures à 20°C, on ajoute 10 ml d'une solution saturée de NaHCO₃ puis successivement on extrait à l'acétate d'éthyle, sèche les phases organiques sur MgSO₄, évapore le solvant et chromatographie le résidu sur gel de silice, éluant : dichlorométhane/méthanol 99/1 (v/v)puis 95/5 ; F = 80°C.

### 5-Ethoxy-1-[4-(N-tert-butylcarbamoyl)-2-méthoxybenzènesulfonyl]-3-spiro-[4-(2-tosyloxyéthyloxy)cyclohexane]indolin-2-one. Composé (II A.3)

De la même manière que pour la préparation du composé *(II A.2)*, à partir de 5-éthoxy-1-[4-(2-hydroxyéthyloxy)cyclohexane]indolin-2-one ou par réaction du chlorure de 4-(N-*tert*-butylcarbamoyl)-2-méthoxybenzènesulfonyle sur le composé *(III.4)* dans les conditions décrites pour la préparation du composé *(II A. 1)*, on isole le produit attendu ; F = 142°C.

### PREPARATION 6 Alcools de formule (II'A)

### 5-Ethoxy-3-spire-[4-(2-hydroxyéthyloxy)cyclohexane]-1-[4-(N-tert-butyl carbamoyl-2-méthoxybenzènesulfonyl)indolin-2-one. Composé (II'A. 1)

a) 5-Ethoxy-3-spire-[4-(2-nitrooxyéthyloxy)cyclohexane]-1-[4-(N*-tert*-butylcarbamoyl-2-méthoxybenzènesulfonyl)indolin-2-one. *Composé (II' A. 1)*
   On chauffe à reflux durant 48 heures un mélange de 0.6 g de *composé (II A. 1)*, 0,8 g de nitrate d'argent et 0,25 g d'iodure de sodium dans 10 ml d'acétonitrile. On sépare les sels par filtration et on évapore les solvants. On isole le produit attendu par chromatographie sur gel de silice en éluant avec un mélange cyclohexane/acétate d'éthyle 80/20 (v/v) ; F = 80°C (hydrate).
b) On chauffe à reflux durant une heure 0,5 g du nitrate précédent, 0,5 ml de cyclohexène, 0,5 g de palladium sur charbon à 10% dans 15 ml d'éthanol puis on sépare le catalyseur par filtration, on évapore le solvant et on chromatographie le résidu sur gel de silice en éluant au dichlorométhane puis avec un mélange dichlorométhane/méthanol 99/1(v/v). On isole le mélange d'isomères du produit attendu ; F = 120°C (hémihydrate) puis l'isomère polaire qui est cristallisé dans un mélange d'éther isopropylique et d'acétate d'éthyle (1/1 ; v/v) ; F = 189°C (hydrate).

### 5-Ethoxy-3-spiro-[4-(3-hydroxypropyloxy)cyclohexane]-1-[4-(N-tert-amyl carbamoyl)-2-méthoxybenzènesulfonyl]indolin-2-one. Composé (II'A.2)

a) 5-Ethoxy-3-spire-[4-(3-méthoxyméthyloxypropyloxy)cyclohexane]-1-[4-(N-*tert*-amylcarbamoyl)-2-méthoxybenzènesulfonyl]indolin-2-one.
   On condense selon le mode opératoire décrit dans la PREPARATION 5 le 5-éthoxy-3-spiro-[4-(3-méthoxyméthyloxypropyloxy)cyclohexane]indolin-2-one *(III'.2P)* de la PREPARATION 10 avec le chlorure de N-*tert*-amylcarbamoyl-2-méthoxysulfonyle pour obtenir le produit attendu qui est engagé tel quel dans l'étape suivante.
b) On chauffe à 50°C durant une heure un mélange de 0,5 g du *composé* préparé en a) dans 1,5 ml de méthanol et 0,2 ml d'acide chlorhydrique concentré (36%). On ajoute 5 ml d'eau, on extrait à l'acétate d'éthyle, puis on évapore les solvants puis on isole le produit attendu après chromatographie sur gel de silice en éluant avec un mélange de cyclohexane/acétate d'éthyle 1/1 (v/v) ; F = 120°C.

### PREPARATION 7 Indolin-2-one de formule (II.b)

### 5-Chloro-3-spiro-[4-(2-morpholinoéthyloxy)cyclohexane]indolin-2-one (mélange d'isomères). Composé (II B. 1)

On chauffe durant 24 heures à 85°C un mélange de 0,57 g de *composé (III.3)*, 0,5 g de morpholine et 0,27 g de Nal dans 6 ml de diméthylformamide. On ajoute 10 ml d'eau au mélange réactionnel et 10 ml d'une solution saturée de NaHCO₃ puis successivement on extrait deux fois à l'acétate d'éthyle, sèche les phases organiques sur MgSO₄, évapore le solvant et chromatographie le résidu sur gel de silice en éluant au dichlorométhane puis avec un mélange dichlorométhane/méthanol 98/2 (v,v) pour isoler 0.5 g du produit attendu sous forme d'huile.
¹H RMN : 10.4 (s, 1H) ; 7,4 (s, 1H) ; 7,2 (d, 1H) ; 6,8 (d, 1H) ; 3,6 (m, 7H) ; 2,4 (m, 6H) ; 1,9-1,6 (m, 8H).

### 5-Ethoxy-3-spire-[4-(2-N-tert-butyloxycarbonyl-N-(benzyloxycarbonylméthyl) amino)éthyloxy)cyclohexane]indolin-2-one (mélange d'isomères). Composé (II B.2)

On chauffe à 60°C pendant 48 heures 1,5 g du tosylate *(III.4)* (mélange d'isomères), 0,66 g de chlorhydrate de glycinate de benzyle et 0,35 g de carbonate de sodium dans 80 ml d'acétonitrile. On évapore le solvant sous pression réduite, reprend le résidu avec 40 ml d'acétate d'éthyle, lave la phase organique à l'eau, sèche sur Na₂SO₄ et évapore le solvant On chromatographie le résidu sur gel de silice en éluant avec un mélange dichlorométhane/méthanol 99/1 (v/v) et isole une résine qui est solubilisée dans 20 ml de dioxane. On ajoute à 5°C, 0,13 g de MgO et 0,539 g de di-*tert*-butyldicarbonate en solution dans 10 ml de dioxane et on agite pendant 16 heures à 20°C. On évapore le solvant, reprend le résidu par de l'acétate d'éthyle, lave la phase organique successivement avec une solution tampon pH = 2, une solution saturée de bicarbonate de sodium et à l'eau. On sèche sur Na₂SO₄ et évapore le solvant. Après purification par chromatographie sur gel de silice en éluant avec un mélange acétate d'éthyle/cyclohexane 5/5 (v/v), on obtient le produit attendu sous forme d'une résine.
¹H RMN :10,12 (s, 0,3H) ; 10,03(s,0,7H) ; 7,30 (m, 5H) ; 6,88 (d, 1H) ; 6,70 (d, 2H) ; 5,14 (s, 0.7H) ; 5,12 (s, 0,3H) ; 4,05 (m, 2H) ; 3,95 (q, 2H) ; 3,3 à 3,6 (m, 5H) ; 1,4 à 2,1 (m, 8H) ; 1,2 à 1,4 (m, 12H).

### 5-éthoxy-3-spiro-[4-(2-(N-tert-butyloxycarbonylamino)éthyloxy)cyclohexane] indolin-2-one. Composé (II B.3)

a) 5-éthoxy-3-spiro-[4-(2-aminoéthyloxy)cyclohexane]indolin-2-one.
   On chauffe à 50°C pendant 16 heures un mélange de 1,5 g du composé *(III.4)* (obtenu à partir de l'isomère polaire *(III'.1)*, 0,23 g d'azoture de sodium dans 15 ml de diméthylformamide. On ajoute 30 ml d'eau, extrait deux fois à l'acétate d'éthyle. On sèche les phases organiques sur Na₂SO₄, évapore partiellement le solvant sous pression réduite jusqu'à un volume d'environ 20 ml. Cette solution est hydrogénée à 60°C sous une pression de 10⁶ Pa en présence de 0,6 g de catalyseur de Lindlar (Palladium sur CaCO₃). On filtre le catalyseur et évapore le solvant sous pression réduite. Le résidu est chromatographié sur une colonne de gel de silice en éluant avec un mélange dichlorométhane/méthanol 90/10 (v/v). On isole après recristallisation de la base dans de l'acétate d'éthyle puis chlorhydratation dans l'acétate d'éthyle le chlorhydrate hydrate du produit attendu ; F = 168°C.
b) A 0,27 g du composé précédent dans 20 ml de dioxane, on ajoute successivement vers +5°C, 0,4 ml d'hydroxyde de sodium 2N, 0,05 g d'oxyde de magnésium et 0,19 g de di-*tert*-butyldicarbonate en solution dans 7 ml de dioxane. Après 2 heures d'agitation à 20°C, on évapore le solvant puis reprend le résidu par 10 ml d'acétate d'éthyle, lave la phase organique successivement avec une solution tampon pH = 2, une solution saturée de bicarbonate de sodium et à l'eau. On sèche sur Na₂SO₄, évapore le solvant et isole le produit attendu sous forme d'une résine.
   ¹H RMN: 10,02 (s, 1H) ; 6,91 (s, 1H) ; 6,68 (s, 2H) ; 3,92 (q, 2H) ; 3,55-3,35 (m, 3H) ; 3,05 (m, 2H); 2,05-1,45 (m, 8H) ; 1,36 (s, 9H) ; 1,27 (t, 3H) ;

### PREPARATION 8 Hydrazides de formule (VII)

### N'-(4-Ethoxyphényl)-4,4-éthylènedioxycyclohexane)carbohydrazide. Composé (VII.1)

On ajoute à -40°C, 1,65 ml de chloroformiate d'isobutyle à un mélange de 2,63 g de 4,4-éthylènedioxycyclohexanoate de sodium dans 20 ml de tétrahydrofurane puis 1,8 ml de triéthylamine. On agite le mélange réactionnel durant 2 heures à 0°C, puis on ajoute à -20°C, 2,4 g de chlorhydrate de 4-éthoxyphénylhydrazine, on agite le mélange réactionnel durant 2 heures à 0°C puis on ajoute 100 ml d'eau et on extrait à l'acétate d'éthyle. Les phases organiques sont lavées successivement à l'eau, avec une solution de KHSO₄ (pH 2), avec une solution saturée de carbonate de potassium, séchées sur MgSO₄ et évaporées. On obtient le produit attendu après cristallisation dans l'éther diéthylique ; F = 158°C.

### N'-phényl-4,4-éthylènedioxycyclohexanecarbohydrazide. Composé (VII.2)

De la même manière, on isole le composé *(VII.2)* à partir de la phénylhydrazine. F = 158°C.

### PREPARATION 9 Acétals de formule (IV')

### 5-Ethoxy-3-spiro-(4,4-éthylènedioxycyclohexane)indolin-2-one. Composé (IV'.1)

A -50°C, on ajoute 2,15 ml d'une solution de butyllithium 1,6 M dans l'hexane à une suspension de 1 g de l'hydrazide *(VII.1)* dans 16 ml de tétrahydrofurane. On agite le mélange réactionnel durant 15 minutes et on ajoute 16 ml de tétraline. On distille le tétrahydrofurane et on chauffe à 180°C durant 45 minutes. On ajoute alors à à température ambiante 20 ml d'acétate d'éthyle, puis successivement on lave à l'eau, sèche la phase organique sur MgSO₄, distille les solvants sous vide et on chromatographie le résidu sur gel de silice en éluant avec un mélange cyclohexanelacétate d'éthyle 7/3 (v/v). On isole le produit attendu par cristallisation dans l'éther diéthylique ; F = 183°C

Le même produit est également obtenu par réaction de 5-éthoxy-3-spiro-(4-oxocyclohexane)indolin-2-one *(composé V.1)* avec l'éthylèneglycol dans le cyclohexane en présence de tamis moléculaire 5 Å et d'acide paratoluènesulfonique en quantité catalytique.

### 5-Ethoxy-3-spiro-(4,4-propylènedioxycyclohexane)indolin-2-one. Composé (IV'.2)

On procède selon le même mode opératoire décrit précédemment pour la préparation du *composé (IV'.1)* à partir de l'hydrazide correspondant ou par réaction de 5-éthoxy-3-spiro-(4-oxocyclohexane)indolin-2-one (*composé V.1*) avec du 1,3-propanediol dans le cyclohexane en présence de tamis moléculaire 5 Å et d'acide paratoluènesulfonique en quantité catalytique ; F = 216°C.

### 3-Spiro-(4,4-éthylènedioxycyclohexane)indolin-2-one. Composé (IV'.3)

On procède selon le mode opératoire décrit précédemment pour la préparation du *composé (IV'1)* à partir de l'hydrazide *(VII.2)* correspondant ; F = 218°C.

### PREPARATION 10 Alcools de formule (III') et (III' P)

### 5-Ethoxy-3-spiro-[4-(2-hydroxyéthyloxy)cyclohexane]indolin-2-one. Composé (III'. 1)

A 0°C, on ajoute lentement 20,2 ml d'une solution de borohydrure de zinc 0,25 M dans l'éther diéthylique (préparée selon la méthode décrite dans Chem. Pharm. Bull., 1984, *32* (4), 1411-1415) à 3,1 g d'acétal *IV'.1* dans 20 ml de dichlorométhane puis 2,8 ml de chlorure de triméthylsilane. On agite le mélange réactionnel durant 16 heures à 20°C puis on ajoute 20 ml d'une solution saturée de NaHCO₃, et successivement on évapore les solvants, extrait à l'acétate d'éthyle, sèche sur MgSO₄, évapore le solvant et on purifie le résidu par chromatographie sur gel de silice en éluant avec un mélange cyclohexane/acétate d'éthyle 67/34 (v/v). On isole le mélange d'isomères du produit attendu puis l'isomère polaire qui est cristallisé dans l'éther diéthylique ; F = 125°C.

### 5-Ethoxy-3-spiro-[4-(3-hydroxypropyloxy)cyclohexane]indolin-2-one. Composé (III'.2)

On procède selon le même mode opératoire que précédemment pour la préparation du *composé (III'.1)* à partir de l'acétal *(IV'.2)*, on obtient l'isomère polaire du produit attendu ; F = 180°C (hémihydrate).

### 5-Ethoxy-3-spiro-[4-(3-méthoxyméthyloxypropyloxy)cyclohexane]-indolin-2-one. Composé (III'.2 P)

On agite durant 24 heures à température ambiante une solution de 1 g de 5-éthoxy-3-spiro-[4-(3-hydroxypropyloxy)cyclohexane]indolin-2-one *(III'.2), 7,7* ml de diméthoxyméthane, 0,065 g de LiBr et 0,07 g d'acide paratoluènesutfonique dans 15 ml de dichlorométhane et on ajoute 10 ml d'une solution saturée de NaCI. On sépare et sèche la phase organique sur MgSO₄, et distille le solvant pour obtenir l'isomère polaire du produit attendu après chromatographie sur gel de silice en éluant avec un mélange de cyclohexane/acétate d'éthyle 1/1 (v/v) ; F = 89°C.

### PREPARATION 11 Alcools protégés de formule (X)

### 5-Ethoxy-3-spiro-(4-méthoxyméthyloxycyclohexane)-1-[(4-N-tert-butylcarbamoyl-2-méthoxybenzènesulfonyl]indolin-2-one. Composé (X. 1)

On ajoute 0,283 g de *tert*-butylate de potassium à une solution refroidie à -40°C de 5-éthoxy-3-spiro-(4-méthoxyméthyloxycyclohexane)indolin-2-one, *(composé de formule XI)* préparé selon EP 636608, dans 80 ml de tétrahydrofurane. On laisse remonter la température à 0°C puis refroidit le mélange à -40°C et ajoute 0,73 g de chlorure de (2-méthoxy-4-N-*tert*-butylcarbamoyl)benzènesulfonyle dans 7 ml de tétrahydrofurane. On agite le mélange réactionnel pendant 2 heures à température ambiante, puis successivement on ajoute 20 ml d'eau , extrait à l'acétate d'éthyle, sèche sur MgSO₄, évapore le solvant et purifie l'huile obtenue par chromatographie sur gel de silice en éluant avec un mélange cyclohexane/acétate d'éthyle 8/2 (v/v). On isole l'isomère le moins polaire du produit attendu ; F = 165°C puis l'isomère polaire ; F = 156°C.

### PREPARATION 12 Alcools de formule (IIc)

### 5-Ethoxy-3-spiro-(4-hydroxycyclohexane)-1-[(4-N-tert-butylcarbamoyl-2-méthoxybenzènesulfonyl]indolin-2-one. Composé (IIC. 1)

On chauffe à 50°C durant 1 heure un mélange de l'isomère polaire du *composé X.1* dans 1,2 ml de méthanol et 0,24 ml d'acide chlorhydrique concentré (36 %). On ajoute 8 ml d'eau au mélange réactionnel, puis successivement on extrait au dichiorométhane, sèche les phases organiques sur MgSO₄ et on évapore les solvants. On obtient le produit attendu après purification par chromatographie sur gel de silice en éluant au dichlorométhane ; F = 268°C. (isomère polaire)

De la même manière, à partir de l'isomère le moins polaire préparé selon (*X.1*), on isole l'isomère le moins polaire du produit attendu ; F = 130°C (hémihydrate). *Composé (IIc.2)*

### PREPARATION 13 Réactifs de formule (2)

### Chlorure de 2-méthoxy-4-N-tert-amylcarbamoylbenzènesulfonyle. Réactif (2).1

a) N-*tert*-amyl(3-méthoxy-4-nitro)benzamide
   On ajoute à 10°C 30 ml de *tert-*amytamine à une solution de 27 g de chlorure de 3-méthoxy-4-nitrobenzoyle (obtenu à partir de 25 g d'acide correspondant et de chlorure de thionyle à reflux durant 4 heures suivi d'une évaporation sous vide) dans 250 ml de dichlorométhane. On agite le mélange réactionnel durant 30 minutes à 20°C, puis on ajoute 100 ml d'une solution d'acide chlorhydrique 1N, décante, lave et sèche la phase organique sur MgSO₄, puis on évapore le solvant et on chromatographie le résidu sur gel de silice en éluant au dichlorométhane pour obtenir 31 g du produit attendu ; F = 65°C.
   De la même manière et à partir de N-*tert*-butylamine, on prépare le N-*tert*-butyl (3-méthoxy-4-nitro)benzamide; F = 118°C.
b) N*-tert*-amyl(3-méthoxy-4-amino)benzamide
   On chauffe à reflux durant 3 heures un mélange de 31 g de N-*tert*-amyl(3-méthoxy-4-nitro)benzamide obtenu en a), 20 g de palladium sur charbon à 10 %, 76 ml de cyclohexane dans 310 ml d'éthanol. On filtre, évapore le filtrat pour obtenir 25 g du produit attendu ; F = 108°C.
   De la même manière, à partir du composé N-*tert*-butyl(3-méthoxy-4-nitro)benzamide on prépare le N-*tert*-butyl-(3-méthoxy-4-amino)benzamide ; F = 160°C.
c) Chlorure de 2-méthoxy-4-*tert*-amylcarbamoylbenzènesulfonyle
   On ajoute à 0°C une solution de 7,9 g de nitrite de sodium dans 31 ml d'eau à une solution de 25 g de N-*tert*-amyl(3-méthoxy-4-amino)benzamide dans 103 ml d'acide acétique et 187 ml d'acide chlorhydrique à 36 %. On agite le mélange réactionnel durant 1 heure à 0°C puis on ajoute cette solution conservée à 0°C à une suspension de 6,8 g de chlorure cuivrique, dans 25 ml d'eau et 140 ml d'acide acétique saturée à 0°C par environ 69 g de dioxyde de soufre. On agite le mélange réactionnel à 0°C durant 3 heures puis à 20°C pendant 16 heures et on coule le milieu sur 750 g de glace en agitant ensuite pendant 1 heure à 20°C. On essore puis successivement on rince le précipité à l'eau, sèche sous vide durant 48 heures pour obtenir 19 g du produit attendu ; F = 104°C

### Chlorure de 4-N-tert-butylcarbamoyl-2-méthoxybenzènesulfonyle. Réactif (2).2

De la même manière, à partir de N-*tert*-butyl(3-méthoxy-4-amino)benzamide, on isole le réactif attendu ; F = 148°C.

### Chlorure de 3-méthoxy-4-benzyloxycarbonylbenzènesulfonyle. Réactif (2).3

En utilisant la même réaction que précédemment et, à partir de l'ester benzylique de l'acide 4-amino-3-méthoxybenzoïque (F = 72°C issu de la réduction du dérivé nitré correspondant par l'étain en milieu chlorhydrique ; F = 88°C), on isole le réactif attendu ; F = 55°C.

### N-tert-butyl-4-bromométhyl-3-méthoxybenzamide. Réactif (2).4

On agite à 30°C sous irradiation du spectre visible durant 48 heures un mélange de 3 g de N-*tert*-butyl-4-méthyl-3-méthoxybenzamide, 2,4 g de N-bromosuccinimide, 0,16 g de peroxyde de benzoyle dans 40 ml de tétrachlorure de carbone. On évapore le solvant, puis successivement on ajoute 25 ml d'eau, extrait à l'éther diéthylique, sèche sur MgSO₄, évapore le solvant et chromatographie le résidu sur gel de silice en éluant avec un mélange cyclohexane/acétate d'éthyle 8/2 (v/v). On isole le réactif attendu après cristallisation dans l'éther isopropylique ; F = 114°C.

### EXEMPLE 1

### 5-Ethoxy-1-[4-(N-tert-butylcarbamoyl))-2-méthoxybenzènesulfonyl]-3-spiro-[4-(2-morpholinoéthyloxy)cyclohexane]indolin-2-one

(I) : R₁ = 5-OC₂H₅ ; R₂ = H ; R₃ = 2-OCH₃ ; W = SO₂ ;
R₄ = 4-CONHC(CH₃)₃ ; 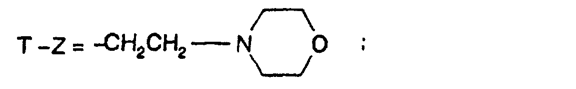 isomère le moins polaire.

Sous atmosphère inerte, on chauffe à 60°C durant 40 heures un mélange de 0,6 g de dérivé chloré *(II A.1)* obtenu selon la PREPARATION V. 0,26 g de morpholine, 0,15 g d'iodure de sodium dans 6 ml de diméthylformamide. On évapore le solvant sous vide, puis successivement on reprend avec 20 ml d'une solution aqueuse de NaHCO₃ à 5%, extrait à l'acétate d'éthyle, lave les phases organiques avec une solution de NaCI à 10%, sèche sur MgSO₄, évapore le solvant et on isole une résine qui est chromatographiée sur gel de silice en éluant avec un mélange dichlorométhane/méthanol 98/2 (v, v).

On isole l'isomère le moins polaire du produit attendu (Rf = 0,5 ; CCM silice; dichlorométhane/méthanol 95/5 (v, v)). On prépare le fumarate dans l'acétone et on le cristallise dans l'éther diéthylique ; F = 153°C (EXEMPLE 1).
¹H RMN, DMSO-d6 200 MHz : 8,0 (m, 2H) ; 7,5 (m, 2H) ; 7,4 (s, 1H) ; 6,88 (d, 1H) ; 6,82 (s, 1H) ; 6,6 (s, 2H ; acide fumarique) ; 4,0 (q, 2H) ; 3,6 (s, 3H) ; 3,55 (m, 7H) ; 2,45 (m, 6H) ; 2-1,4 (m, 8H) ; 1,34 (s, 9H) ; 1,3 (t, 3H).

### EXEMPLE 2 5-Ethoxy-1-[4-(N-tert-butylcarbamoyl))-2-méthoxybenzènesulfonyl]-3-spiro-[4-(2-morpholinoéthyloxy)cyclohexane]indolin-2-one

(I) : R₁ = 5-OC₂H₅ ; R₂ = H ; R₃ = 2-OCH₃ ; W = SO₂ ;
R₄ = 4-CONHC(CH₃)₃ ; 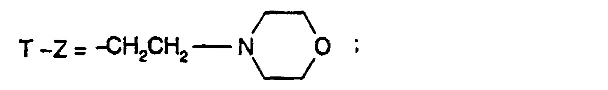 isomère le plus polaire.

On isole l'isomère le plus polaire du produit préparé ci-dessus selon l'EXEMPLE 1 dans les conditions précédentes ; Rf = 0,43 ; F = 212°C-216°C.
¹H RMN, DMSO-d6 200 MHz : 8,0 (m, 2H) ; 7,5 (m. 2H) ; 7,4 (s, 1H) ; 7,03 (s; 1H) ; 6,84 (d, 1H); 6,6 (s, 2H ; acide fumarique) ; 4,0 (q, 2H) ; 3,6 (s, 3H) ; 3.5 (m, 6H) ; 3,40 (m, 1H) ; 2,45 (m, 6H) ; 1,9-1,6 (m, 8H) ; 1,34 (s, 9H) ; 1,3 (t, 3H).

On prépare le fumarate dans l'acétone et on le cristallise dans l'éther diéthylique ; F = 172°C (EXEMPLE 2).

On prépare le dihydrogénophosphate monohydraté par réaction de l'acide phosphorique monohydraté sur la base dans l'éthanol ; F = 170°C. On prépare le nitrate par réaction de l'acide nitrique aqueux sur la base dans l'éthanol ; F = 155°C.

### EXEMPLE 3

### 5-Ethoxy-1-[4-(N',N'-diéthyluréido)-2-méthoxybenzènesulfonyl]-3-spiro-[4-(2-diméthylaminoéthyloxy)cyclohexane]indolin-2-one.

(I) : R₁ = 5-OC₂H₅ ; R₂ = H : R₃ = 2-OCH₃ ; W = SO₂

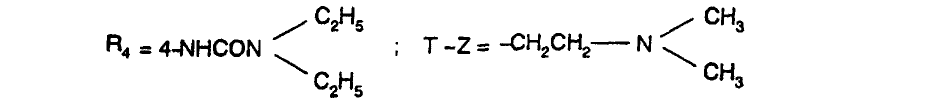

On agite à 20°C durant 48 heures un mélange de 0,23 g du dérivé tosylé (*II A.2*) obtenu précédemment selon la PREPARATION 5 dans 3,3 ml d'acétonitrile et 0,23 ml d'une solution aqueuse de diméthylamine à 40%. On ajoute 1 ml d'une solution saturée de NaHCO₃, et successivement on extrait à l'acétate d'éthyle, sèche sur MgSO₄, évapore le solvant, chromatographie le résidu sur gel de silice en éluant avec un mélange de dichlorométhane/méthanol/ammoniaque (245/5/0,2 v/v/v) ; (Rf = 0,5 ; CCM silice ; dichlorométhane/méthanol/ammoniaque 85/15/1 v/v/v) ; F = 103°C.

### EXEMPLE 4

### 5-Ethoxy-3-spiro-[4-(2-aminoéthyloxy)cyclohexane]-1-[4-(4-N-tert-butylcarbamoyl)-2-méthoxybenzènesulfonyl]indolin-2-one (mélange d'isomères).

(I) : R₁ = 5-OC₂H₅ ; R₂ = H ; R₃ = 2-OCH₃ ; W = SO₂ ;
R_{4 = 4-}CONHC(CH₃)₃ ; T -Z = -CH₂CH₂NH₂
a) 5-Ethoxy-3-spiro-[4-(2-azidoéthyloxy)cyclohexane]-1-[4-(4-N-*tert*butyl-carbamoyl)-2-méthoxybenzènesulfonyl]indolin-2-one. (mélange d'isomères)
   Sous atmosphère inerte on chauffe à 100°C durant 2 heures un mélange de 0,5 g de dérivé chloré *II A. 1* obtenu précédemment selon la PREPARATION 5, 0,06 g d'azoture de sodium et 0,126 g d'iodure de sodium dans 5 ml de diméthytformamide. On ajoute 10 ml d'eau au mélange réactionnel puis on extrait à l'acétate d'éthyle et successivement on lave les phases organiques à l'eau, sèche sur Na₂SO₄ on concentre partiellement le solvant jusqu'à un volume de 20 ml pour obtenir une solution d'azide qui est utilisée telle quelle dans la réaction suivante.
b) On hydrogène la solution obtenue en a) à 40°C durant 60 heures sous 10⁶Pa en présence de 0,2 g de palladium/CaCO₃ (catalyseur de Lindlar ; 5% Pd). On sépare le catalyseur par filtration, évapore le solvant et chromatographie sur une colonne de gel de silice en éluant avec un mélange dichlorométhane/méthanol 8/2 (v/v). On isole le produit attendu sous forme de base que l'on salifie par l'acide fumarique dans l'acétone et cristallise dans l'éther isopropylique pour obtenir le produit attendu ; F = 138°C (monohydrate).

De la même manière, à partir du *composé (II A.3)* et par le même enchaînement, on isole l'isomère polaire du produit attendu dont le chlorhydrate trihémihydraté fond à 174°C.

### EXEMPLE 5

### 5-Chloro-3-spiro-[4-(2-morpholinoéthyloxy)cyclohexane]-1-[4-(N-tert-butylcarbamoyl)-2-méthoxybenzènesulfonyl]indolin-2-one.

(I) : R₁ = 5-Cl;R₂ = H;R₃ = 2-OCH₃ ; W = SO₂
R₄=4-CONHC(CH₃)₃ ;

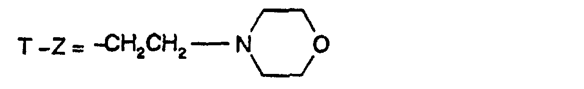

On ajoute 0,073 g de *tert*-butylate de potassium à une solution refroidie à -30°C de 0,21 g du *composé II B.1* obtenu précédemment selon la PREPARATION 7 dans 24 ml de tétrahydrofurane. On laisse remonter la température à 0°C puis on refroidit le mélange à -40°C et on ajoute 0,19 g de chlorure de [2-méthoxy-4-(N-*tert*-butylcarbamoyl)]benzènesulfonyle dans 2 ml de tétrahydrofurane. On agite ensuite le mélange réactionnel pendant 2 heures à -10°C, on ajoute 15 ml d'eau, puis successivement on extrait à l'acétate d'éthyle, sèche sur MgSO₄, évapore le solvant et purifie le résidu par chromatographie sur gel de silice en éluant au dichlorométhane puis avec un mélange dichlorométhane/méthanol 96/4. On isole l'isomère polaire du produit attendu qui est salifié par l'acide fumarique dans l'acétone. Le fumarate est cristallisé dans l'éther diisopropylique ; F = 107°C (trihémihydrate).

### EXEMPLE 6

### 5-Ethoxy-3-spiro-[4-(2-carboxyéthyloxy)cyclohexane]-1-[4-(N-tert-amylcarbamoyl)-2-méthoxybenzènesulfonyl]indolin-2-one.

(I) : R₁ = 5-OC₂H₅ ; R₂ = H ; R₃ = 2-OCH₃ ; W = SO₂ 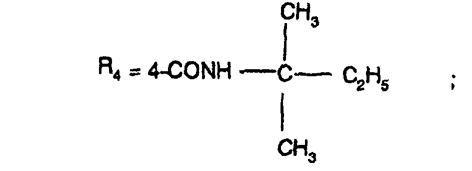 T -Z = -CH₂CH₂―COOH

A 0°C on ajoute 1 g d'oxyde chromique à un mélange de 1,5 g du *composé (II' A.2)* obtenu selon la PREPARATION 6 dans 9 ml d'acide acétique et 10 ml d'eau. On agite le mélange réactionnel pendant deux heures à 20°C, puis on ajoute 80 ml d'eau et successivement on extrait à ('acétate d'éthyle, sèche sur MgSO₄ les phases organlques, distille le solvant et isole le produit attendu après chromatographie sur gel de silice en éluant avec un mélange dichlorométhane/méthanol 99/1 (v/v) ; F = 108°C (hémihydrate).

### EXEMPLE 7

### 5-Ethoxy-3-spiro-(4-éthoxycarbonylméthyloxycyclohexane)-1-[(4-N-tert-butylcarbamoyl-2-méthoxy)benzènesulfonyl]indolin-2-one.

(I): R₁ = 5-OC₂H₅;R₂ = H;R₃ = 2-OCH₃; W=SO₂
R₄₌₄₋CONHC(CH₃)₃ ; T-Z = -CH₂-COO-C₂H₅

On ajoute à 0°C à une solution de 0,75 g de 5-éthoxy-3-spiro-(4-hydroxycyclohexane)-1-[4-(N-*tert*-butylcarbamoyl)-2-méthoxybenzène sulfonyl)indoline-2-one (II.C₁) dans 30 ml de dichlorométhane, 0,47g de 2,6-di-*tert*-butylpyridine, 0.54 g de trifluorométhanesulfonate d'argent puis 0,27 ml d'iodoacétate d'éthyle. On agite le mélange réactionnel durant 48 heures à 20°C, puis successivement on filtre, évapore le solvant, et on isole le produit attendu après chromatographie sur gel de silice en éluant au cyclohexane puis avec un mélange cyclohexane/dichlorométhane 20/80 (v/v) et recristallisation dans l'isopropanol ; F = 165°C.

### EXEMPLE 8

### 5-Ethoxy-3-spiro-(4-carboxyméthyloxycyclohexane)-1-(4-N-tert-butyl carbamoyl-2-méthoxybenzènesulfonyl)indolin-2-one.

(I) : R₁ = 5-OC₂H₅ ; R₂ = H ; R₃ = 2-OCH₃ ; W = SO₂ ;
R₄ = 4-CONHC(CH₃)₃ ; T - Z = -CH₂COOH

On chauffe à 65°C pendant 16 heures 0,34 g du produit obtenu à l'EXEMPLE 7 et 0,01 g d'acide paratoluènesulfonique dans 3 ml d'alcool benzylique. On évapore le solvant puis successivement on ajoute 1 ml d'eau et 1 ml d'une solution saturée de NaHCO₃, extrait à l'acétate d'éthyle, évapore le solvant puis on ajoute 5 ml d'isopropanol, 0,25 g de palladium sur charbon à 10 % et 0,25 ml de cyclohexène. On chauffe le mélange réactionnel à 80°C pendant 3 heures puis successivement on filtre, rince le catalyseur au chlorure de méthylène, évapore les solvants, isole le produit attendu et le purifie par chromatographie sur gel de silice en éluant avec un mélange dichlorométhane /méthanol 98/2 (v/v). On recristallise la fraction du produit attendu dans un mélange d'éther isopropylique/acétate d'éthyle 8/2 (v/v) ; F = 175°C (hémihydrate)

En procédant selon les EXEMPLES 1 à 8 ci-dessus, on prépare les EXEMPLES 9 à 23 décrits dans le TABLEAU 1 ci-après.

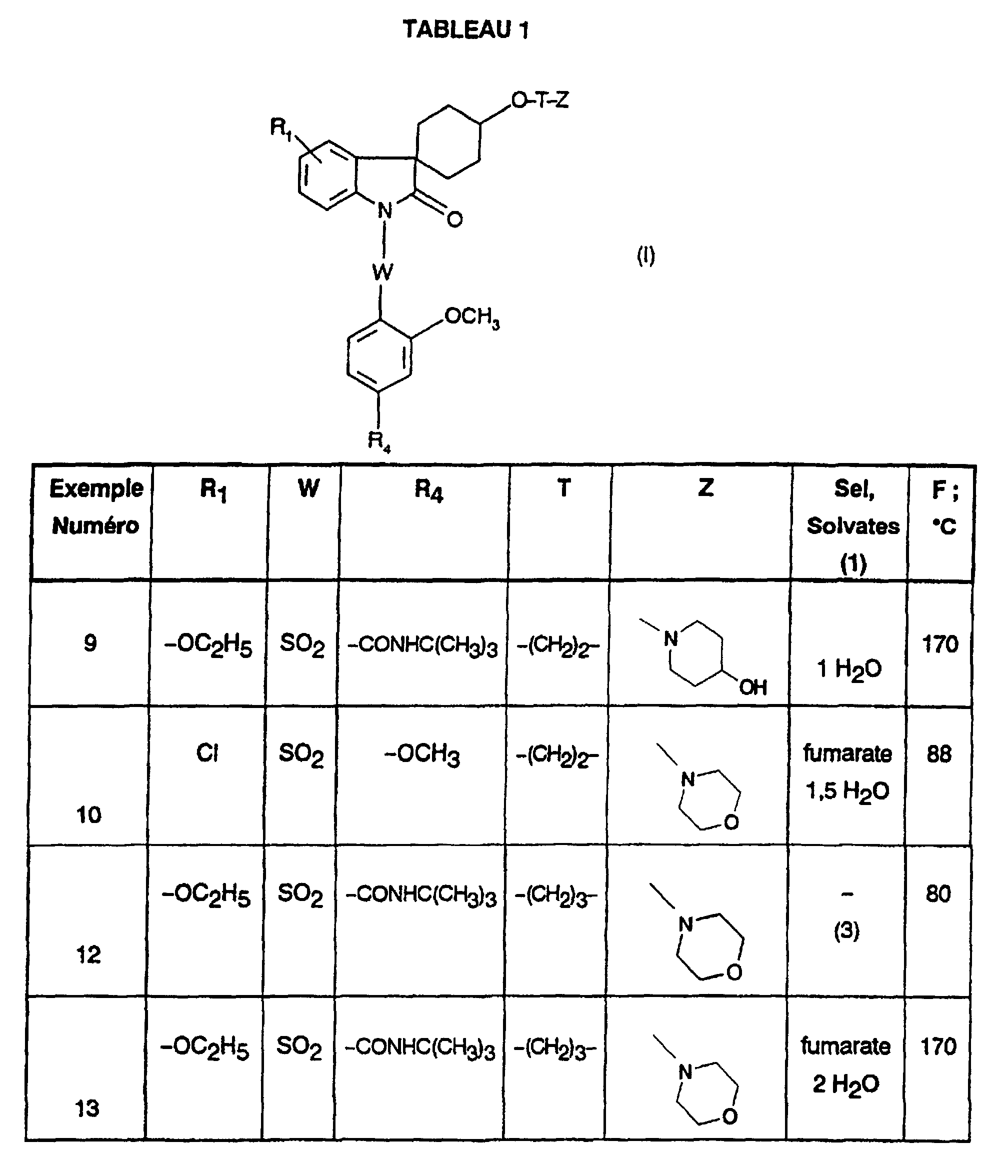

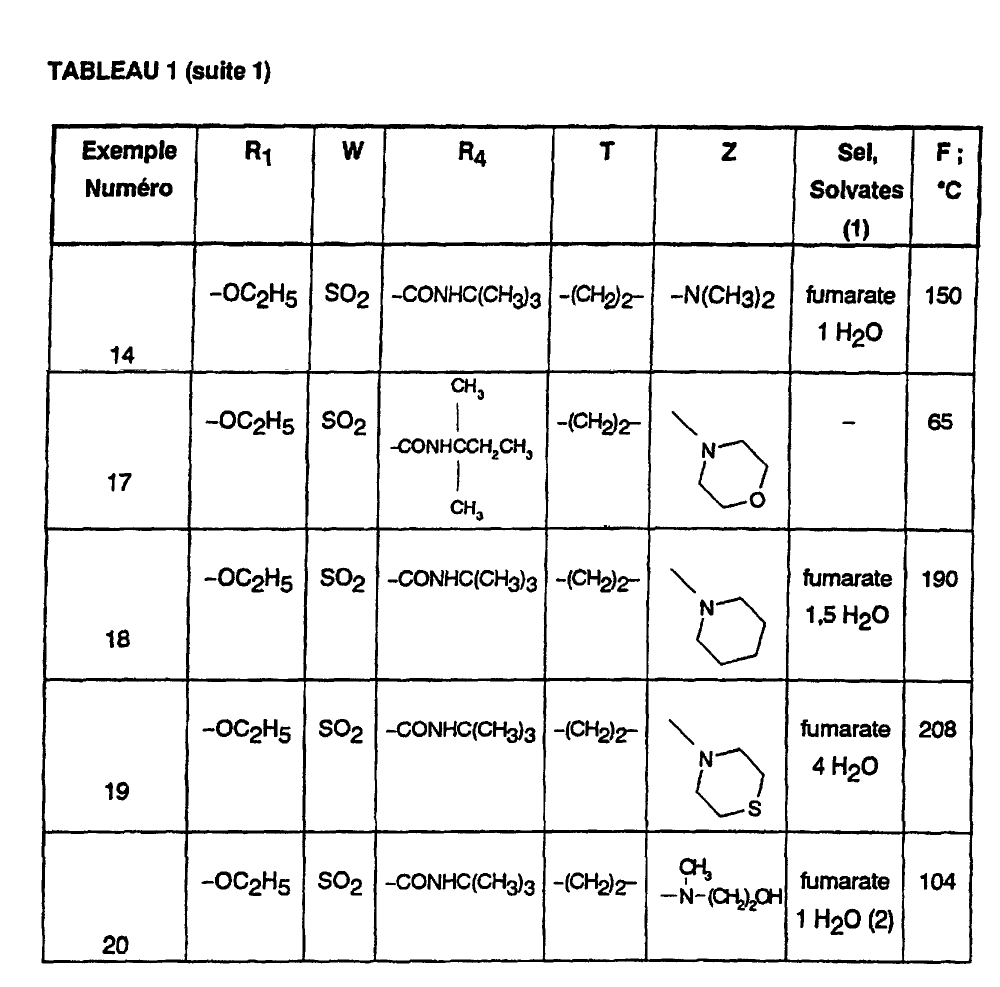

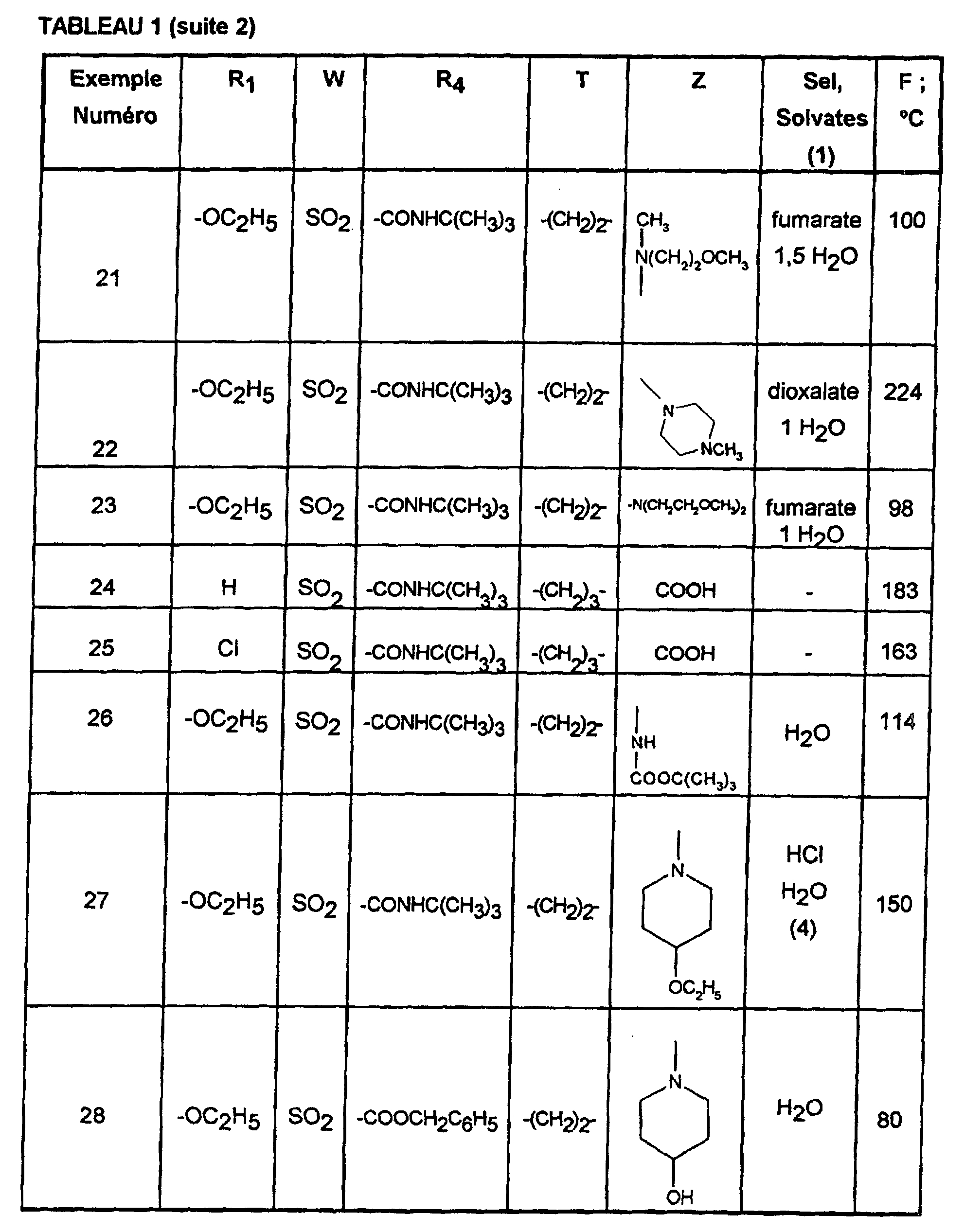

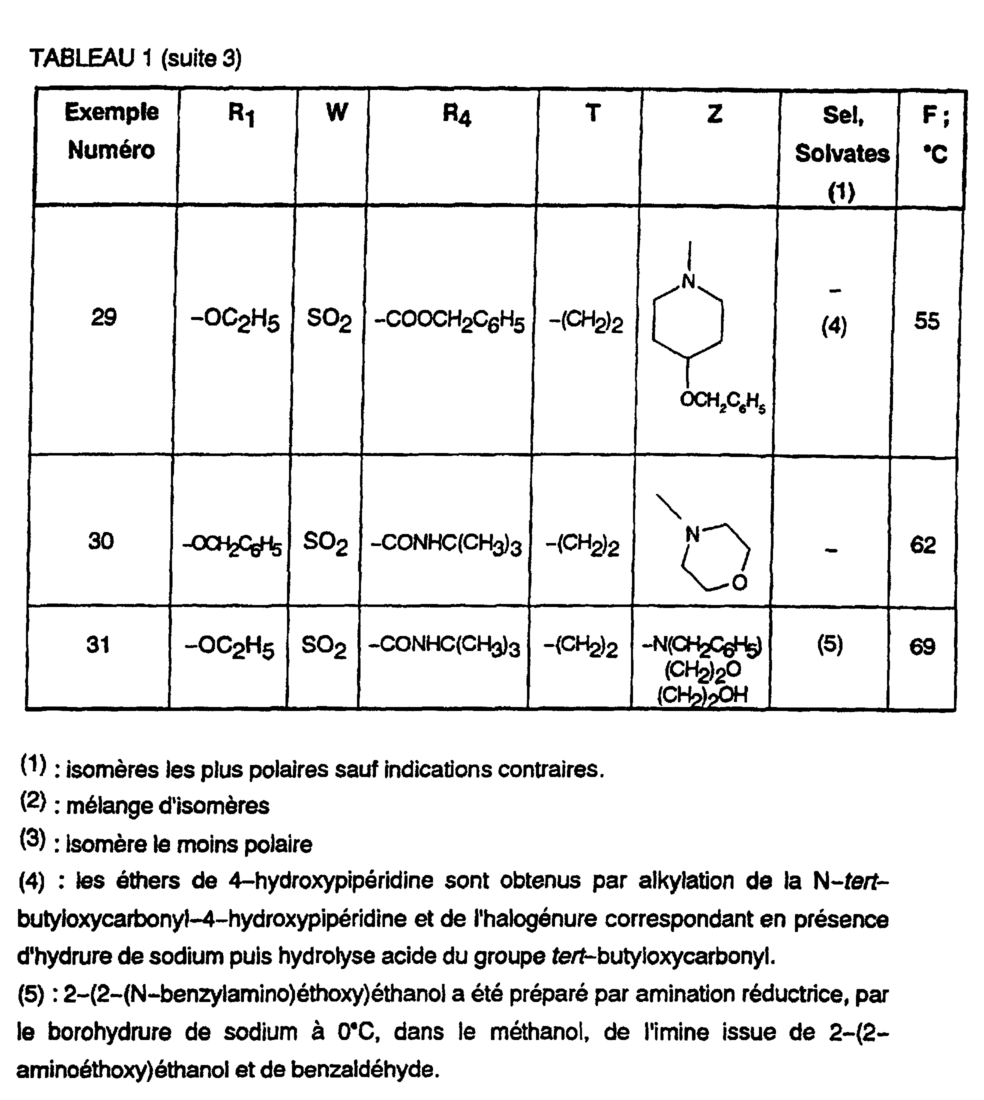

### EXEMPLE 32

### 5-Ethoxy-3-spiro-[4-(2-(2-hydroxyéthylamino)éthyloxy)cyclohexane]-1-[4-(4-N-tert-butylcarbamoyl)-2-méthoxybenzènesulfonyl]indolin-2-one (isomère polaire).

(I):R₁ = 5-OC₂H₅; R₂ = H; R₃ = 2-OCH₃; W = SO₂;
R₄ = 4-CONHC(CH₃)₃; T-Z = CH₂CH₂NHCH₂CH₂OH;
a) On ajoute à une solution refroidie à 5°C de 0,9 g du chlorhydrate de l'amine de l'EXEMPLE 4 (isomère polaire) dans 8 ml de tétrahydrofurane 0,33 g de benzyloxyacétaldéhyde puis 0,46 g de triacétoxyborohydrure de sodium. On agite le mélange réactionnel à 20°C pendant 3 heures, ajoute 10 ml d'HCI 1N, extrait à l'acétate d'éthyle, lave la phase organique avec une solution saturée de NaCl, sèche sur MgSO₄ et évapore le solvant sous pression réduite. On chromatographie le résidu sur une colonne de gel de silice en éluant avec un mélange de dichlorométhane/ méthanol 98/2 (v/v).
b) A l'éther benzylique obtenu précédemment solubilisé dans 5 ml d'acide acétique glacial on ajoute 0,4 ml de 1,4-cyclohexadiène, 0,3 g de Palladium/C (10 %) et chauffe à 60°C sous bullage d'azote durant 16 heures selon la méthode décrite dans J. Org. Chem. *43*, 21 (1978).

On filtre le catalyseur, ajoute 10 ml d'eau au milieu réactionnel, on le neutralise avec une solution saturée de NaHCO₃, extrait à l'acétate d'éthyle, lave à l'eau, sèche sur MgSO₄ et évapore le solvant sous pression réduite. Le résidu est chromatographié sur une colonne de gel de silice en éluant avec un mélange dichlorométhane/méthanol 98/2 (v/v). On isole le produit attendu sous forme de chlorhydrate hydrate par chlorhydratation par une solution d'isopropanol chlorhydrique et cristallisation dans l'éther diéthylique ; F = 130°C.

### EXEMPLE 33

### 5-Ethoxy-3-spiro-[4-(2-(2-(2-hydroxyéthyloxy)éthylamino)éthyloxy)cyclohexane]-1-[4-(4-N-tert-butylcarbamoyl)-2-méthoxybenzènesulfonyl]indolin-2-one.

Par débenzylation du composé de l'EXEMPLE 31 selon le mode opératoire décrit dans l'EXEMPLE 32b) dans l'éthanol et chlorhydratation dans l'éther diéthylique, on isole le composé attendu sous forme de chlorhydrate trihémihydraté ; F = 159°C.

### EXEMPLE 34

### 5-Ethoxy-3-spiro-[4-(2-(4-benzyloxypipéridino)éthyloxy)cyclohexane]-1-[4-carboxy-2-méthoxybenzènesulfonyl]indolin-2-one.

(I): R₁ = 5-OC₂H₅; R₂ = H; R₃ = 2-OCH₃; W = SO₂;
R₄ = 4-COOH; 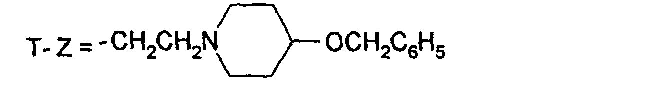 (préparé par débenzylation sélective selon Tetrah. Letters, 1986, 3753).

On ajoute 0,62 ml de *tert*-butyldiméthylsilane et 0,06 ml de triéthylamine à une solution de 0,03 g d'acétate de palladium dans 4 ml de dichlorométhane et agite le mélange réactionnel 15 minutes à 20°C. On ajoute lentement une solution de 1 g du composé décrit dans l'EXEMPLE 29 dans 2,6 ml de dichlorométhane et agite 4 heures à 20°C. On ajoute 1 ml d'acide acétique, filtre, rince au dichlorométhane et lave le filtrat avec une solution aqueuse de chlorure d'ammonium puis à l'eau. On isole le produit attendu après évaporation du solvant, cristallisation dans le pentane et séchage à 50°C sous vide pendant 5 heures ; F = 120°C.

### EXEMPLE 35

### 5-Ethoxy-3-spiro-[4-(2-(4-benzyloxypipéridino)éthyloxy)cyclohexane]-1-[4-(N-(1-hydroxyméthyl)cyclopentylcarbamoyl-2-méthoxybenzènesulfonyl]indolin-2-one.

(I): R₁ = 5-OC₂H₅; R₂ = H; R₃ = 2-OCH₃; W = SO₂;

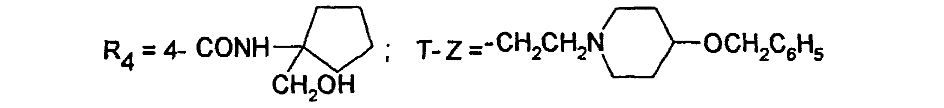

A une suspension de 0,7 g du composé préparé dans l'EXEMPLE 34 dand 7 ml de toluène et 2,5 ml de dichlorométhane on ajoute 1,27 g de chlorure d'oxalyle et on agite le mélange réactionnel pendant 6 heures à 20°C. On évapore les solvants, sèche le résidu 2 heures à 20°C sous vide et le dissout dans 20 ml de toluène puis ajoute cette solution à une solution refroidie vers -40°C de 1,16 g de 1-amino-1-cyclopentaneméthanol dans 30 ml de toluène. On agite 2 heures à 20°C, ajoute 30 ml d'eau et 100 ml d'acétate d'éthyle. La phase organique est séchée sur Na₂SO₄ et évaporée sous pression réduite. On isole le produit attendu après une chromatographie sur gel de silice en éluant avec un mélange dichlorométhane/ méthanol 95/5 (v/v) ; F = 103°C.

### EXEMPLE 36

### 5-Ethoxy-3-spiro-[4-(2-(4-hydroxypipéridino)éthyloxy)cyclohexane]-1-[4-(N-(1-hydroxyméthyl)cyclopentylcarbamoyl-2-méthoxybenzènesulfonyl]indolin-2-one.

(I): R₁ = 5-OC₂H₅; R₂ = H; R₃ = 2-OCH₃; W = SO₂;

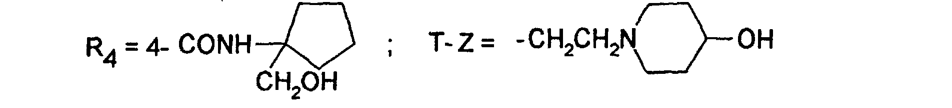

En procédant selon le mode opératoire décrit dans l'EXEMPLE 32 b) à partir du composé de l'EXEMPLE 35 on isole le produit attendu sous forme de base hydratée après chromatographie sur une colonne de gel de silice en éluant avec un mélange dichlorométhane/méthanol 92/8 (v/v) ; F = 109°C.

### EXEMPLE 37 ne fait pas partie de l'invention

### 5-Ethoxy-3-spiro-[4-(2-(benzyloxycarbonylméthylamino)éthyloxy)cyclohexane]-1-[4-(4-N-tert-butylcarbamoyl)-2-méthoxybenzènesulfonyl]indolin-2-one. (mélange d'isomères)

(I): R₁ = 5-OC₂H₅; R₂ = H; R₃ = 2-OCH₃; W = SO₂;
R₄ = 4-CONHC(CH₃)₃ ; T-Z = -CH₂CH₂NHCH₂COOCH₂C₆H₅

En procédant selon le mode opératoire décrit dans l'EXEMPLE 5 à partir du composé *(II B.2)* et de chlorure de 2-méthoxy-4-(N-*tert*-butylcarbamoyl)benzènesulfonyle, on isole le résidu qui est agité pendant deux heures à 20°C dans 3 ml d'une solution d'acétate d'éthyle saturée en acide chlorhydrique gazeux. On obtient le produit attendu après alcalinisation et chromatographie sur gel de silice en éluant avec un mélange cyclohexane/acétate d'éthyle 8/2 (v/v) ; le chlorhydrate monohydraté fond à 160°C.

### EXEMPLE 38 ne fait pas partie de l'invention

### 5-Ethoxy-3-spiro-[4-(2-(carboxyméthylamino)éthyloxy)cyclohexane]-1-[4-(4-N-tert-butylcarbamoyl)-2-méthoxybenzènesulfonyl]indolin-2-one. (mélange d'isomères)

(I): R₁ = 5-OC₂H₅; R₂ = H; R₃ = 2-OCH₃: W = SO₂;
R₄ = 4-CONHC(CH₃)₃ ; T-Z = -CH₂CH₂NHCH₂COOH

On chauffe à reflux durant 1 heure 30 0,06g du composé de l'EXEMPLE 37, 6 g de cyclohexène, 0,05 g de Palladium/charbon à 10 % dans 10 ml d'éthanol, on filtre le catalyseur et évapore le solvant sous pression réduite. Le composé attendu est isolé sous forme dihydratée après une chromatographie sur gel de silice en éluant avec un mélange dichlorométhane/méthanol 90/10 (v/v) ; F = 199°C.

### EXEMPLE 39

### 5-Hydroxy-1-[4-(N-tert-butylcarbamoyl)-2-méthoxybenzènesulfonyl]-3-spiro-[4-(2-morpholinoéthyloxy)cyclohexane]indolin-2-one. (mélange d'isomères)

(I): R₁ = 5-OH; R₂ = H; R₃ = 2-OCH₃; W = SO₂;
R₄ = 4-CONHC(CH₃)₃ ;

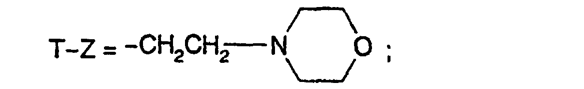

En procédant selon le mode opératoire décrit dans l'EXEMPLE 38 à partir du composé de l'EXEMPLE 30, on isole le produit attendu sous forme hydratée ; F=125°C.

### EXEMPLE 40

### 5-Ethoxy-1-[4-(N-tert-butylcarbamoyl)-2-méthoxybenzènesulfonyl]-3-spiro-[4-(2-N-oxyde morpholinoéthyloxy) cyclohexane]indolin-2-one.

(I): R₁ = 5-OC₂H₅; R₂ = H; R₃ = 2-OCH₃; W = SO₂;
R₄ = 4-CONHC(CH₃)₃ ;

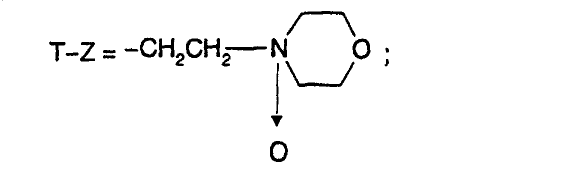

On ajoute 0,8 ml de peroxyde d'hydrogène à 30 % à 0,5 g du composé décrit dans l'EXEMPLE 2 dissout dans 10 ml de méthanol et chauffe le mélange réactionnel à 45°C pendant 16 heures. On évapore le solvant sous pression et chromatographie le résidu sur gel de silice en éluant avec un mélange dichlorométhane/méthanol 85/15 (v/v). Le produit attendu est isolé sous forme hémihydratée après une recristallisation dans un mélange cyclohexane/acétate d'éthyle 40/60 (v/v) ; F = 189°C.

### EXEMPLE 41

### Méthylsulfate de 5-Ethoxy-1-[4-(N-tert-butylcarbamoyl)-2-méthoxybenzènesulfonyl]-3-spiro-[4-(2-N-méhylmorpholiniuméthyloxy)cyclohexane]indolin-2-one.

(I): R₁ = 5-OC₂H₅; R₂ = H; R₃ = 2-OCH₃; W = SO₂;
R₄=4-CONHC(CH₃)₃;

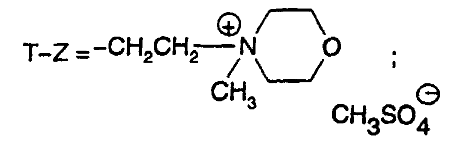

On ajoute 0,05 ml de diméthylsulfate à 0,25 g du composé décrit dans l'EXEMPLE 2 dissout dans 2,5 ml d'acétonitrile et on chauffe le mélange réactionnel à 60°C pendant 24 heures. On évapore le solvant et on isole le produit attendu sous forme hémihydratée après une cristallisation dans l'éther diéthylique et séchage à 40°C sous vide pendant 5 heures ; F = 190°C.

### EXEMPLE 42 ne fait pas partie de l'invention

### 5-Ethoxy-3-spiro-[4-(2-(2-(N-tert-butoxycarbonylglycyl)amino)éthyloxy)cyclohexane]-1-[4-(4-N-tert-butylcarbamoyl)-2-méthoxybenzènesulfonyl]indolin-2-one.

(I): R₁ = 5-OC₂H₅; R₂ = H; R₃ = 2-OCH₃; W = SO₂;
R₄ = 4-CONHC(CH₃)₃; T-Z = -CH₂CH₂-NHCOCH₂NHCOOC(CH₃)₃

A une solution de 0.11 g de N*-α-tert*-butyloxycarbonylglycine dans 2 ml d'acétonitrile, on ajoute à 5°C 0,28 g d'hexafluorophosphate de benzotriazole-1-yl-oxy-tris-(diméthylamino)-phosphonium et 0,24 ml de triéthylamine puis 0,35 g de chlorhydrate du composé de l'EXEMPLE 4 (isomère polaire) et agite vers 20°C pendant 4 heures.

On évapore le solvant sous pression réduite, reprend à l'acétate d'éthyle et lave successivement avec une solution tampon KHSO₄/K₂SO₄ pH = 2, à l'eau, avec une solution saturée de NaHCO₃ puis à l'eau. On sèche la phase organique sur MgSO₄ et évapore le solvant sous pression réduite et chromatographie le résidu sur une colonne de gel de silice en éluant avec un mélange de dichlorométhane/méthanol 99/1 (v/v). On isole le produit attendu ; F = 158°C.

### EXEMPLE 43

### 5-Chloro-3-spiro-[4-(N-(3-diméthylaminopropyl)carbamoylméthoxy)cyclohexane]-1-[4-(4-N-tert-butylcarbamoyl)-2-méthoxybenzènesulfonyl]indolin-2-one.

(I): R₁ = 5-CI; R₂ = H; R₃ = 2-OCH₃; W = SO₂;
R₄ = 4-CONHC(CH₃)₃ ; T-Z = -CH₂CONH(CH₂)₃N(CH₃)₂

En procédant selon le mode opératoire décrit dans l'EXEMPLE 42 et en partant de l'acide carboxylique de l'EXEMPLE 25 et de 3-diméthylaminopropanamine, on isole le composé attendu sous forme de chlorhydrate monohydraté ; F = 135°C.

En procédant selon les modes opératoires des EXEMPLES 42 et 43, on prépare par réactions d'amines ou d'acides convenablement choisis les composés 44 à 50 rassemblés dans le TABLEAU 2 ci-après.

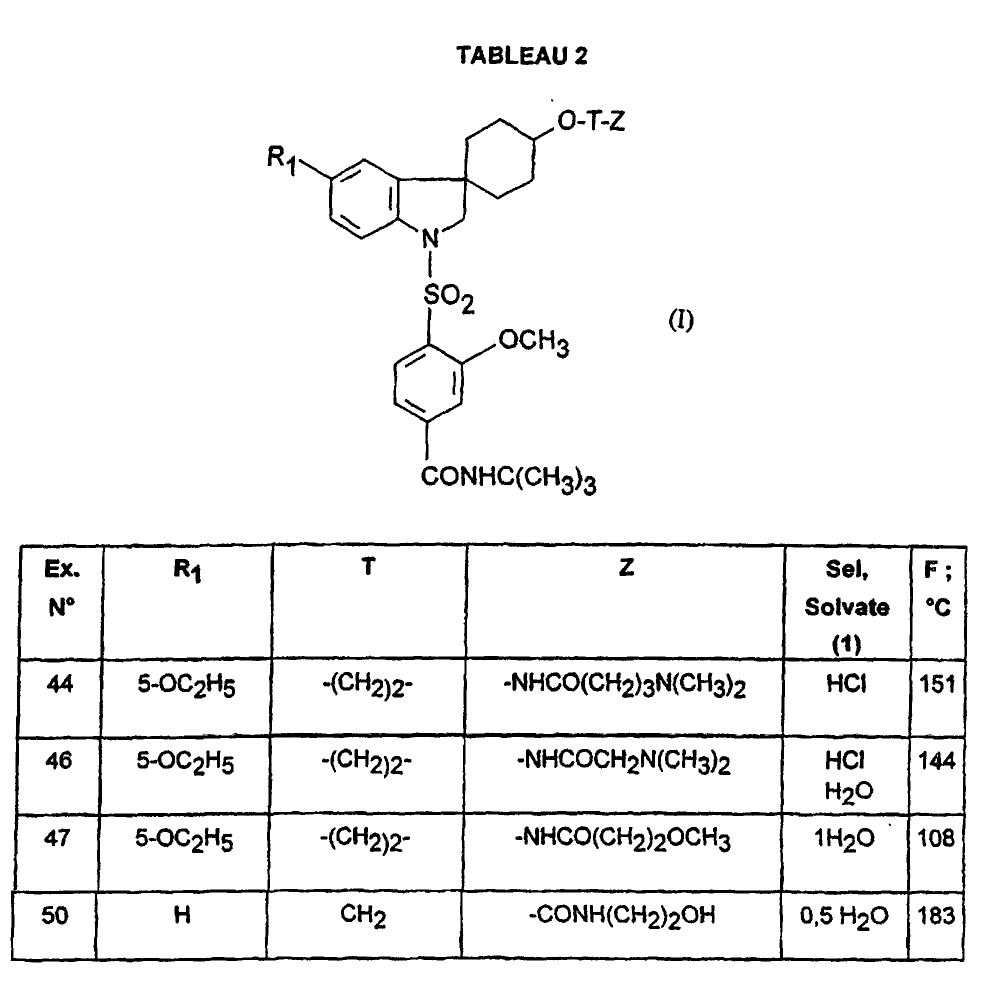

### EXEMPLE 51

### 5-Ethoxy-3-spiro-[4-(2-glycylaminoéthyloxy)cyclohexane]-1-[4-(4-N-tert-butylcarbamoyl)-2-méthoxybenzènesulfonyl]indolin-2-one.

(I): R₁ = 5-OC₂H₅; R₂ = H; R₃ = 2-OCH₃; W = SO₂;
R₄ = 4-CONHC(CH₃)₃ ; T-Z = -CH₂CH₂NHCOCH₂NH₂

A 5°C, on ajoute 3 ml d'une solution saturée d'acide chlorhydrique gazeux dans l'acétate d'éthyle à une suspension de 0,3 g du composé de l'EXEMPLE 42 dans 3 ml d'acétate d'éthyle et on agite pendant 2 heures à température ambiante. On évapore le solvant, on cristallise dans l'éther diéthylique, sèche sous vide pour obtenir le produit attendu sous forme de chlorhydrate dihydraté ; *F* = 169°C.

## Revendications

1. Composé de formule 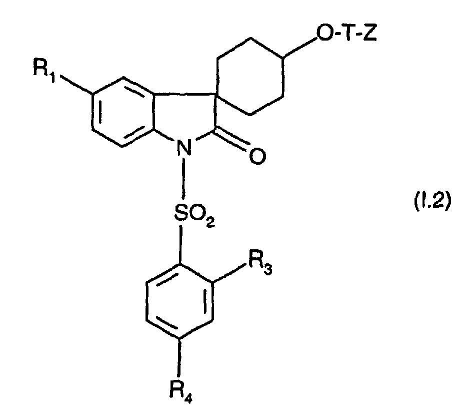 dans laquelle :
- R₁ représente un hydrogène ; un hydroxyle ; un halogène ; un (C₁-C₇)alkyle ; un (C₁-C₇)polyfluoroalkyle ; un (C₁-C₇)alcoxy ; un (C₁-C₇)alkylthio ; un (C₁-C₇)polyfluoroalcoxy ; un (C₃-C₇)cycloalkyloxy ; un (C₃-C₇)cycloalkylthio ; un cycloalkylméthoxy ou un cycloalkylméthylthio dans lesquels le cycloalkyle est en C₃-C₇ ; un phénoxy ; un benzyloxy ; un nitro ; un cyano ;
- R₃ et R₄ représentent chacun indépendamment l'un de l'autre un hydrogène ; un halogène ; un (C₁-C₇)alkyle ; un (C₂-C₇)alcényle ; un (C₁-C₇)polyhalogénoalkyle ; un phényle ou un benzyle ; un cyano ; un nitro ; un groupe -NR₅R₆ ; un hydroxyamino; un hydroxyle ; un groupe OR₇; un groupe SR₇; un groupe -COOR₈ ; un groupe -CONR₉R₁₀ ; un groupe -CSNR₉R₁₀ ; l'un au moins des radicaux R₃ et R₄ étant différent de l'hydrogène ;
- R₅ et R₆ représentent chacun indépendamment un hydrogène ; un (C₁-C₇)alkyle ; un (C₂-C₇)alcényle ; un phényle ; un benzyle ; un (C₁-C₇)alkylcarbonyle ; un (C₁-C₇)alkylthiocarbonyle ; un (C₃-C₇)cycloalkylcarbonyle ; un (C₃-C₇)cycloalkylthiocarbonyle ; un benzoyle ; un thiénylcarbonyle ; un furylcarbonyle ; un (C₁-C₇)alkyloxycarbonyle ; un phénoxycarbonyle ; un benzyloxycarbonyle ; un carbamoyle ou un thiocarbamoyle non substitué ou substitué par R₉ et R₁₀; ou bien R₅ et R₆ constituent avec l'atome d'azote auquel ils sont liés un groupe hétérocyclique choisi parmi les groupes pyrrolidine, pyrroline, pyrrolyle, indoline, indole, pipéridine ;
- R₇ représente un (C₁-C₇)alkyle ; un (C₂-C₇)a(cényle ; un phényle ; un benzyle ; un (C₃-C₇)cycloalkyle ; un (C₁-C₇)polyfluoroalkyle ; un formyle ; un (C₁-C₇)alkylcarbonyle ; un benzoyle ; un benzylcarbonyle ;
- R₈ représente un hydrogène, un (C₁-C₇)alkyle ; un phényle ; un benzyle ;
- R₉ et R₁₀ représentent chacun indépendamment l'hydrogène ; un (C₁-C₇)alkyle ; un (C₁-C₇)polyfluoroalkyle ; un (C₂-C₇)alcényle ; un (C₃-C₇)cycloalkyle éventuellement substitué par un groupe hydroxy (C₁-C₄)alkyle ; un pyridyle ; un phényle ; un thiényle ; un furyle ; un (C₄-C₇)azacycloalkyle ; ou bien R₉ et R₁₀ constituent avec l'atome d'azote auquel ils sont liés un groupe hétérocyclique choisi parmi les groupes pyrrolidine, pipéridine et pipérazine, non substitués ou substitués par des (C₁-C₄)alkyles ;
- T représente un (C₁-C₄)alkylène éventuellement interrompu par un (C₃-C₆)cycloalkylène, lesdits alkylènes étant éventuellement substitués une ou plusieurs fois sur le même atome de carbone par un (C₁-C₃)alkyle ; ou bien T représente une liaison directe;
- Z représente un groupe -NR₁₁R₁₂ ; -⁺NR₁₁R₁₂(C₁-C₄)alkyle (A⁻), (A⁻) étant un anion ; -N(O)R₁₁R₁₂ ; un groupe -COOR₁₁ ; un groupe -NR₁₁COR₁₂ ; un groupe -CONR₁₁R₁₂, étant entendu que lorsque T représente un méthylène ou une liaison directe, Z ne peut pas être -NR₁₁R₁₂; -⁺NR₁₁R₁₂(C₁-C₄)alkyle (A⁻); -N(O)R₁₁R₁₂ ; NR₁₁COR₁₂ ;
- R₁₁ et R₁₂ représentent chacun indépendamment l'hydrogène ; un (C₁-C₇)alkyle ; un (C₁-C₄)alcoxy ; un (C₃-C₇)cycloalkyle ; un phényle ; un (C₁-C₃)alkylène-cycloalkyle dans lequel le cycloalkyle est en C₃-C₇; un (C₁-C₃)alkylènephényle; lesdits groupes pouvant éventuellement être mono- ou poly-substitués par R₁₃ ;
ou bien R₁₁ et R₁₂ constituent éventuellement avec l'atome d'azote auquel ils sont liés un hétérocycle choisi parmi les hétérocycles azétidine, pyrrolidine, pipéridine, pipérazine, pipérazinone, morpholine, morpholinone, thiomorpholine, hexahydroazépine, éventuellement mono-substitué par R₁₃ ;
- R₁₃ représente un (C₁-C₄)alkyle ; un hydroxyle ; un hydroxyalkyloxy ; un (C₁-C₄)alcoxy; un groupe -NR₁₄R₁₅ dans lequel R₁₄ et R₁₅ représentent chacun indépendamment l'hydrogène ou un (C₁-C₄)alkyle ; un amidino ; un guanidino ; un imidazolyle ; un pyridyle ; un indolyle ; un tétrahydroisoquinolyle ;
- le groupe phényle constitutif des substituants R₁, R₃, R₄, R₅, R₆, R₇, R₈, R₉, R₁₀, R₁₁ et R₁₂ étant non substitué, mono- ou di-substitué par un (C₁-C₇)alkyle, un (C₁-C₇)alkoxy, un trifluorométhyle, un halogène ou trisubstitué par un (C₁-C₇)alkyle, un (C₁-C₇)alkoxy ou un halogène;
ainsi que leurs sels, solvates où hydrates.

2. Composé selon la revendication 1 de formule : 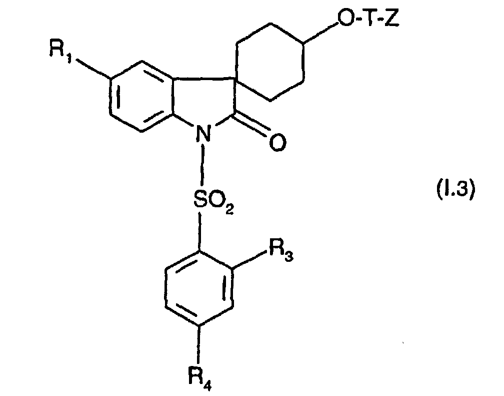 dans laquelle R₁, R₃ et R₄ sont tels que définis pour (I.2) dans la revendication 1, T représente un (C₁-C₃)alkylène et Z représente un groupe amino, un 2-hydroxyéthylamino, un 2-(2-hydroxy)éthyloxyéthylamino, un morpholinyle ou un carboxy, et ses sels, solvates ou hydrates.

3. Composé selon la revendication 1 de formule : 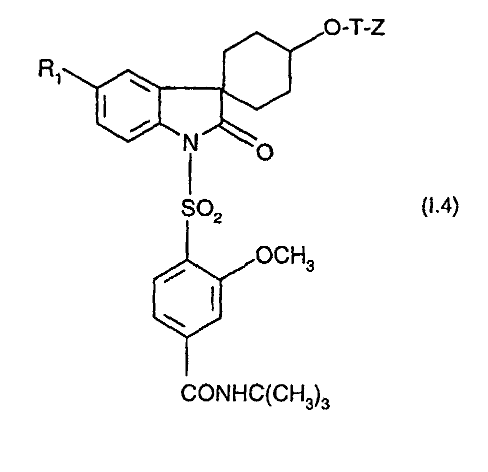 dans laquelle R₁, T et Z sont tels que définis pour (I.2) dans la revendication 1, ou un de ses sels, solvates ou hydrates.

4. Composé de formule : 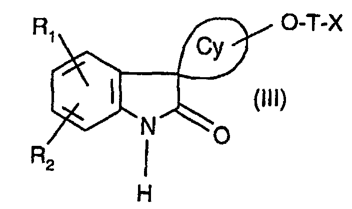 dans laquelle :
- Cy constitue avec le carbone auquel il est lié un cyclohexane substitué en position 4 par -O-T-X ;
- R₂ est un atome d'hydrogène ;
- R₁ et T sont tels que définis pour (I.2) dans la revendication 1 ; et
- X est un halogène, un mésyloxy ou un tosyloxy ;
- ou bien X représente un groupe azido,
ou un de ses sels, solvates ou hydrates.

5. Composé selon la revendication 4, **caractérisé** en ce qui est l'un des composés ci-après :
* 5-Ethoxy-3-spiro-[4-(3-chloropropyloxy)cyclohexane]indolin-2-one ;
* 5-Ethoxy-3-spiro-[4-(2-chloroéthyloxy)cyclohexane)indolin-2-one ;
* 5-Chloro-3-spiro-[4-(2-chloroéthyloxy)cyclohexane]indolin-2-one ;
* 5-Ethoxy-3-spiro-[4-(2-tosyloxyéthyloxy)cyclohexane]indolin-2-one.

6. Composé selon la revendication 1, **caractérisé en ce qu'**il est l'un des composés ci-après :
* 5-Chloro-3-spiro-[4-(2-morpholinoéthyloxy)cyclohexane]-1-[4-(N-*tert*-butylcarbamoyl)-2-méthoxybenzènesulfonyl]indolin-2-one ;
* 5-Ethoxy-3-spiro-[4-(2-aminoéthyloxy)cyclohexane)-1-[4-(4-N-*tert*-butylcarbamoyl)-2-méthoxybenzènesulfonyl]indolin-2-one ;
* 5-Ethoxy-3-spiro-[4-(2-(N-méthyl-N-(2-hydroxyéthyl)amino) éthyl oxy)cyclohexane]-1-[4-(N-*tert*-butylcarbamoyl)-2-méthoxybenzène sulfonyl]indolin-2-one ;
* 5-Ethoxy-1-[4-(N-*tert*-butylcarbamoyl))-2-méthoxybenzènesulfonyl]-3-spiro-[4-(2-morpholinoéthyloxy)cyclohexane]indolin-2-one ;
* 5-Ethoxy-3-spiro-(4-carboxyméthyloxycyclohexane)-1-(4-N-*tert*-butyl carbamoyl-2-méthoxybenzènesulfonyl)indolin-2-one ;
* 5-Ethoxy-3-spiro-[4-(2-morpholinoéthyloxy)cyclohexane]-1-[4-(N-*tert*-amylcarbamoyl)-2-méthoxybenzènesulfonyl]indolin-2-one ;
* 5-Ethoxy-3-spire-[4-(2-carboxyéthyloxy)cyclohexane]-1-[4-(N-*tert-* amylcarbamoyl)-2-méthoxybenzènesulfonyl]indolin-2-one ;
* 5-Ethoxy-1-[4-(N',N'-diéthyluréido)-2-méthoxybenzènesulfonyl]-3-spiro-[4-(2-diméthylaminoéthyloxy)cyclohexane]indolin-2-one;
* 5-Ethoxy-3-spiro-[4-(2-(4-éthoxypipéridino)éthyloxy)cyclohexane]-1-[4-(N-*tert*-butylcarbamoyl)-2-méthoxybenzènesulfonyl]indolin-2-one ;
* 5-Ethoxy-3-spiro-[4-(2-glycylaminoéthyloxy)cyclohexane]-1-[4-(N*-tert*-butylcarbamoyl)-2-méthoxybenzènesulfonyl]indolin-2-one ;
* 5-Ethoxy-3-spiro-[4-(2-(N,N-diméthylglycylamino)éthyloxy)cyclohexane]-1-[4-(N-*tert*-butylcarbamoyl)-2-méthoxybenzènesulfonyl]indolin-2-one ;
* 5-Chloro-3-spiro-[4-(N-(3-diméthylaminopropyl)carbamoylméthyloxy)cyclohexane]-1-[4-(N-*tert*-butylcarbamoyl)-2-méthoxybenzènesulfonyl]indolin-2-one ;
* 5-Ethoxy-3-spiro-[4-(2-(4-diméthylaminobutyrylamino)éthyloxy)cyclohexane]-1-[4-(N-*tert*-butylcarbamoyl)-2-méthoxybenzènesulfonyl]indolin-2-one ;
* 5-Ethoxy-3-spire-[4-(2-(2-hydroxyéthylamino)éthyloxy)cyclohexane]-1-[4-(N-*tert*-butylcarbamoyl)-2-méthoxybenzènesulfonyl]indolin-2-one;
* 5-Ethoxy-3-spiro-[4-(2-(2-(2-hydroxyéthyloxy)éthylamino)éthyloxy)cyclohexane]-1-[4-(N-*tert*-butylcarbamoyl)-2-méthoxybenzènesulfonyl]indolin-2-one ;
ou leurs sels, solvates ou hydrates pharmaceutiquement acceptables.

7. Composé selon la revendication 1, **caractérisé en ce qu'**il est la 5-éthoxy-1-[4-(N-tert-butylcarbamoyl)-2-méthoxybenzènesulfonyl]-3-spiro-[4-(2-morpholinoéthyloxy)cyclohexane]indoline-2-one, ainsi que ses sels, solvates ou hydrates pharmaceutiquement acceptables.

8. Procédé de préparation d'un composé selon l'une quelconque des revendications 1 à 3, 6 et 7 **caractérisé en ce que** :
(1) soit lorsque Z = NR₁₁R₁₂ dans lequel R₁₁ et R₁₂ sont tels que définis pour (I.2):
(1a) lorsqu'au moins l'un des radicaux R₁₁ et R₁₂ est différent de l'hydrogène, on fait réagir sur un composé de formule 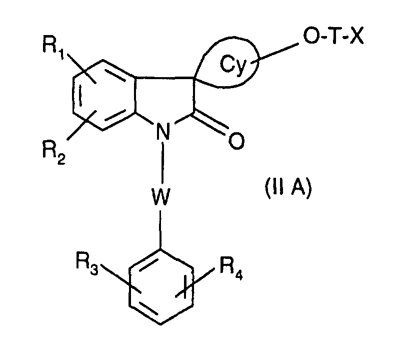 dans lequel :
- Cy constitue avec le carbone auquel il est lié un cyclohexane substitué en position 4 par -O-T-X ;
- R₂ est un atome d'hydrogène ;
- W est SO₂ ;
- R₁, R₃, R₄ et T sont tels que définis pour (I.2) dans la revendication 1 ; et
- X représente un halogène, un mésyloxy ou un tosyloxy,
avec un dérivé de formule ZH dans un solvant choisi parmi le diméthylformamide, le tétrahydrofurane ou l'acétonitrile, à des températures comprises entre 0° et 120°C ;
(1b) lorsque R₁₁ et R₁₂ = H, le composé (IIA) dans lequel X est un azido est réduit en amino ;
(2) soit lorsque Z = -COOH on oxyde un composé de formule : 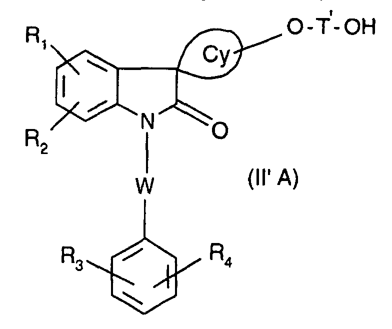 dans laquelle R₁, R₂, W, R₃, R₄ sont tels que définis ci-dessus et Cy constitue avec le carbone auquel il est lié un cyclohexane substitué en position 4 par -O-T'-OH, dans lequel T' représente T-CH₂- dans un solvant acide à une température comprise entre 0°C et 100°C ;
(3) soit on fait réagir un composé de formule : 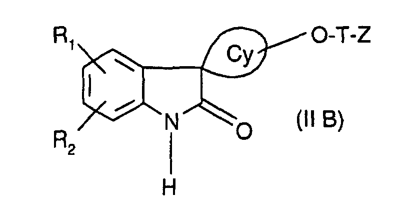 dans lequel R₁, R₂, T et Z sont tels que définis ci-dessus et Cy constitue avec l'atome de carbone auquel il est lié un cyclohexane substitué en position 4 par -O-T-Z, avec un composé de formule : 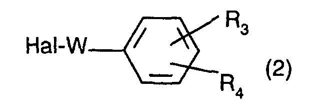 dans lequel W, R₃ et R₄ sont tels que définis ci- dessus et Hal représente un atome d'halogène dans un solvant anhydre en présence d'un hydrure métallique ou d'un alcoolate alcalin à des températures comprises entre -40° et 25°C ;
(4) soit lorsque Z = -COOH on oxyde un composé de formule : 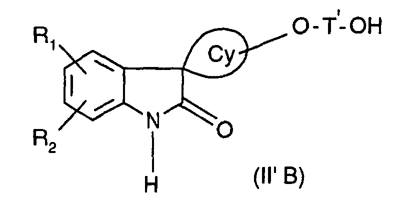 dans laquelle R₁ et R₂ sont tels que définis ci-dessus et Cy constitue avec l'atome de carbone auquel il est lié un cyclohexane substitué en position 4 par -O-T'-OH, dans lequel T' représente T-CH₂ puis ensuite, on protège éventuellement l'acide ainsi obtenu de formule : 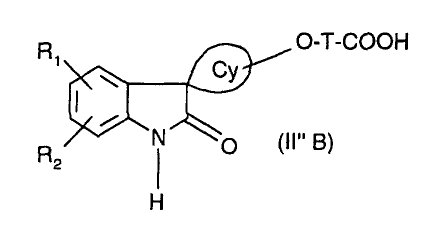 dans lequel R₁, R₂ et T sont tels que définis ci-dessus et Cy constitue avec l'atome de carbone auquel il est lié un cyclohexane substitué en position 4 par -O-T-COOH protégé par un groupement protecteur de l'acide carboxylique pour obtenir l'intermédiaire de formule : 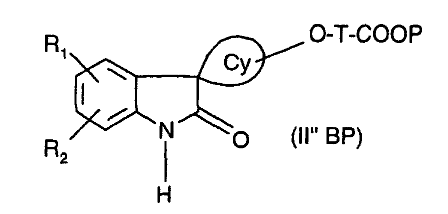 dans lequel R₁, R₂ et T sont tels que définis ci-dessus, Cy constitue avec l'atome de carbone auquel il est lié un cyclohexane substitué en position 4 par -O-T-COOP, dans lequel P représente un groupe protecteur choisi parmi un alkyle, un *tert*-butyle ou un benzyle et on soumet enfin ce composé (II"BP) à l'action d'un dérivé de formule (2), pour obtenir après déprotection un composé (I.2), un de leurs ammoniums quaternaires, oxydes, sulfones ou sels.

9. Composition pharmaceutique contenant à titre de principe actif, un composé de formule (I.2) selon la revendication 1 ou un de ses sels, hydrates ou solvates pharmaceutiquement acceptables.

10. Composition pharmaceutique contenant, en tant que principe actif, un composé de formule (I.3) selon la revendication 2 ou un de ses sels, hydrates ou solvates pharmaceutiquement acceptables.

11. Composition pharmaceutique contenant, en tant que principe actif, un composé de formule (I.4) selon la revendication 3 ou un de ses sels, hydrates ou solvates pharmaceutiquement acceptables.

12. Composition pharmaceutique contenant, en tant que principe actif, un composé selon la revendication 6.

13. Composition pharmaceutique selon une quelconque des revendications 9 à 12 contenant également un autre principe actif.

14. Composition pharmaceutique selon la revendication 13 **caractérisée en ce que** l'autre principe actif est un antagoniste spécifique du récepteur de l'angiotensine II.

15. Composition pharmaceutique selon la revendication 14, **caractérisée en ce que** l'antagoniste spécifique du récepteur de l'angiotensine II est l'irbésartan.

16. Composition pharmaceutique contenant une association de 5-éthoxy-1-[4-(N-*tert*-butylcarbamoyl))-2-méthoxybenzènesulfonyl]-3-spiro-[4-(2-morpholinoéthyloxy)cyclohexane]indolin-2-one et l'irbésartan.

17. Composé selon l'une quelconque des revendications 1 à 3, 6 et 7 pour son utilisation comme médicament.

18. Utilisation d'un composé selon l'une quelconque des revendications 1 à 3, 6 et 7 pour la préparation de médicaments destinés à traiter les maladies vasopressine-dépendantes ou ocytocine-dépendantes.

19. Utilisation d'un composé selon l'une quelconque des revendications 1 à 3, 6 et 7 pour la préparation de médicaments destinés à traiter l'insuffisance cardiaque.

20. Utilisation d'un composé selon l'une quelconque des revendications 1 à 3, 6 et 7 pour la préparation de médicaments destinés à traiter la glaucome.

21. Utilisation d'une composition selon l'une quelconque des revendications 9 à 16 pour la préparation de médicaments destinés à traiter ou à prévenir l'insuffisance cardiaque.

22. Utilisation d'une composition selon l'une quelconque des revendications 9 à 16 pour la préparation de médicaments destinés à traiter ou à prévenir le syndrome de la sécrétion inappropriée de l'hormone antidiurétique (SIADH).

## Claims

1. Compound of formula 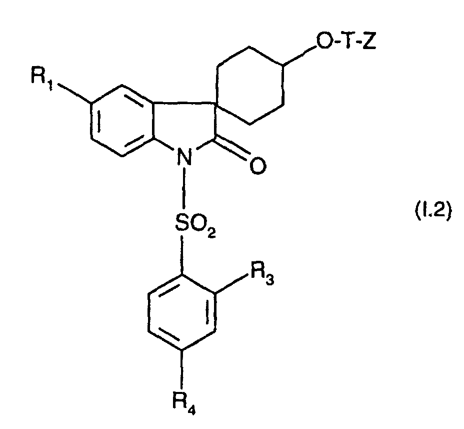 in which:
- R₁ represents a hydrogen; a hydroxyl; a halogen; a (C₁-C₇)alkyl; a (C₁-C₇)polyfluoroalkyl; a (C₁-C₇)alkoxy; a (C₁-C₇)-alkylthio; a (C₁-C₇)polyfluoroalkoxy; a (C₃-C₇)cycloalkyloxy; a (C₃-C₇)cycloalkylthio; a cycloalkylmethoxy or a cycloalkylmethylthio in which the cycloalkyl is C₃-C₇; a phenoxy; a benzyloxy; a nitro; or a cyano;
- R₃ and R₄ represent, independently of one another, a hydrogen; a halogen; a (C₁-C₇)alkyl; a (C₂-C₇)alkenyl; a (C₁-C₇)polyhaloalkyl; a phenyl or a benzyl; a cyano; a nitro; an -NR₅R₆ group; a hydroxyamino; a hydroxyl; an OR₇ group; an SR₇ group; a -COOR₈ group, a -CONR₉R₁₀ group; a -CSNR₉R₁₀ group, at least one of the R₃ and R₄ radicals being other than hydrogen;
- R₅ and R₆ each independently represent a hydrogen; a (C₁-C₇)alkyl; a (C₂-C₇)alkenyl; a phenyl; a benzyl; a (C₁-C₇)alkylcarbonyl; a (C₁-C₇)alkylthiocarbonyl; a (C₃-C₇)cycloalkylcarbonyl; a (C₃-C₇)cycloalkylthiocarbonyl; a benzoyl; a thienylcarbonyl; a furylcarbonyl; a (C₁-C₇)alkyloxycarbonyl; a phenoxycarbonyl; a benzyloxycarbonyl; a carbamoyl or a thiocarbamoyl which is unsubstituted or substituted by R₉ and R₁₀; or alternatively R₅ and R₆ form, with the nitrogen atom to which they are bonded, a heterocyclic group chosen from the pyrrolidine, pyrroline, pyrrole, indoline, indole and piperidine groups;
- R₇ represents a (C₁-C₇)alkyl; a (C₂-C₇)alkenyl; a phenyl; a benzyl; a (C₃-C₇)cycloalkyl; a (C₁-C₇)polyfluoroalkyl; a formyl; a (C₁-C₇)alkylcarbonyl; a benzoyl; or a benzylcarbonyl;
- R₈ represents a hydrogen; a (C₁-C₇)alkyl; a phenyl; or a benzyl;
- R₉ and R₁₀ each independently represent hydrogen; a (C₁-C₇)alkyl; a (C₁-C₇)polyfluoroalkyl; a (C₂-C₇)alkenyl; a (C₃-C₇)cycloalkyl optionally substituted by a hydroxy (C₁-C₄)alkyl; a pyridyl; a phenyl; a thienyl; a furyl; a (C₄-C₇)azacycloalkyl or alternatively R₉ and R₁₀ form, with the nitrogen atom to which they are bonded, a heterocyclic group chosen from the pyrrolidine, piperidine or piperazine groups, which is unsubstituted or substituted by (C₁-C₄)alkyls;
- T represents a (C₁-C₄)alkylene which is optionally interrupted by a (C₃-C₆)cycloalkylene, the said alkylenes optionally being substituted one or a number of times on the same carbon atom by a (C₁-C₃)alkyl; or alternatively T represents a direct bond;
- Z represents an -NR₁₁R₁₂ group; -⁺NR₁₁R₁₂(C₁-C₄)-alkyl (A⁻), (A⁻) being an anion; -N(O)R₁₁R₁₂; a -COOR₁₁ group; an -NR₁₁COR₁₂ group; a -CONR₁₁R₁₂ group; it being understood that when T represents a methylene or a direct bond, Z cannot be -NR₁₁R₁₂; -⁺NR₁₁R₁₂(C₁-C₄)alkyl(A⁻); -N(O)R₁₁R₁₂; -NR₁₁COR₁₂;
- R₁₁ and R₁₂ each independently represent hydrogen; a (C₁-C₇)alkyl; a (C₁-C₄)alkoxy; a (C₃-C₇)cycloalkyl; a phenyl; a (C₁-C₃)alkylenecycloalkyl, in which the cycloalkyl is C₃-C₇; a (C₁-C₃)alkylenephenyl; it being possible for the said groups optionally to be mono- or polysubstituted by R₁₃ ;
or alternatively R₁₁ and R₁₂ optionally form, with the nitrogen atom to which they are bonded, a heterocycle chosen from azetidine, pyrrolidine, piperidine, piperazine, piperazinone, morpholine, morpholinone, thiomorpholine and hexahydroazepine heterocycles, which heterocycle is optionally mono- or polysubstituted by R₁₃;
- R₁₃ represents a (C₁-C₄)alkyl; a hydroxyl group; a hydroxyalkyloxy; a (C₁-C₄)alkoxy; an -NR₁₄R₁₅ group in which R₁₄ and R₁₅ each independently represent hydrogen or a (C₁-C₄)alkyl; an amidino; a guanidino; an imidazolyl; a pyridyl; an indolyl; a tetrahydroisoquinolyl;
- the phenyl group, which is constituent of the R₁, R₃, R₄, R₅, R₆, R₇, R₈, R₉, R₁₀, R₁₁ and R₁₂ substituents, being unsubstituted, mono- or disubstituted by a (C₁-C₇)alkyl, a (C₁-C₇)alkoxy, a trifluoromethyl, a halogen or trisubstituted by a (C₁-C₇)alkyl, a (C₁-C₇)-alkoxy or a halogen;
and its salts, solvates or hydrates.

2. Compound according to claim 1 of formula: 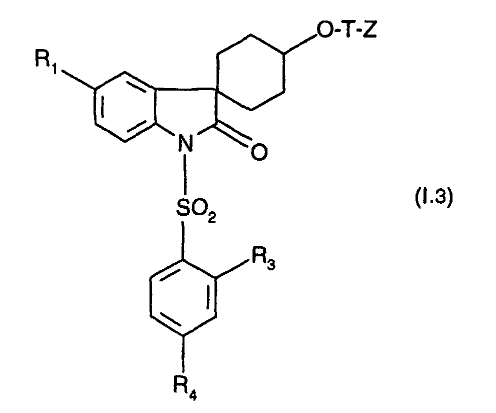 in which R₁, R₃ and R₄ are as defined for (I.2) in claim 1, T represents a (C₁-C₃)alkylene and Z represents an amino group, a 2-hydroxyethylamino, a 2-(2-hydroxy)ethyloxyethylamino, a morpholinyl or a carboxyl, and its salts, solvates or hydrates.

3. Compound according to claim 1 of formula: 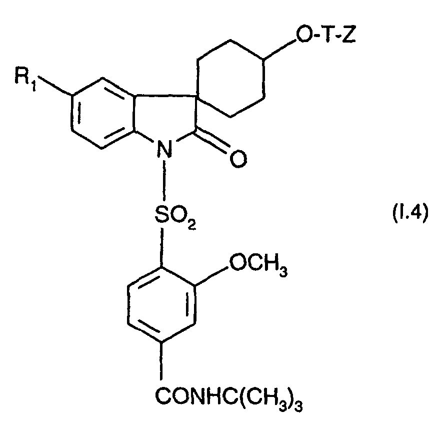 in which R₁, T and Z are as defined for (I.2) in claim 1 or one of its salts, solvates or hydrates.

4. Compound of formula: 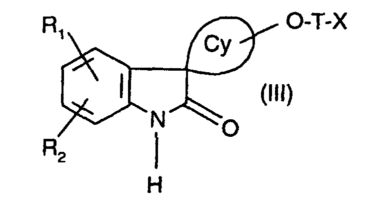 in which:
- Cy forms with the carbon atom to which it is bonded a cyclohexane substituted in 4-position by -O-T-X;
- R₂ represents a hydrogen atom;
- R₁ and T are as defined for (I.2) in claim 1; and
- X is a halogen, a mesyloxy or a tosyloxy;
- or alternatively X represents an azido group,
or one of its salts, solvates or hydrates.

5. Compound according to claim 4, **characterized in that** it is one of the compounds below:
* 5-Ethoxy-3-spiro-[4-(3-chloropropyloxy)cyclohexane]indolin-2-one;
* 5-Ethoxy-3-spiro-[4-(2-chloroethyloxy)cyclohexane]indolin-2-one;
* 5-Chloro-3-spiro[4-(2-chloroethyloxy)cyclohexane]-indolin-2-one;
* 5-Ethoxy-3-spiro-[4-(2-toxyloxyethyloxy)cyclohexane]indolin-2-one.

6. Compound according to claim 1, **characterized in that** it is one of the compounds below:
* 5-chloro-3-spiro-[4-(2-morpholinoethyloxy)cyclohexane]-1-[4-(N-*tert*-butylcarbamoyl)-2-methoxybenzenesulphonyl]indolin-2-one;
* 5-ethoxy-3-spiro-[4-(2-aminoethyloxy)cyclohexane]-1-[4-(4-N-*tert*-butylcarbamoyl)-2-methoxybenzenesulphonyl]indolin-2-one;
* 5-ethoxy-3-spiro-[4-(2-(N-methyl-N-(2-hydroxyethyl)amino)ethyloxy)cyclohexane]-1-[4-(N-*tert*-butylcarbamoyl)-2-methoxybenzenesulphonyl]indolin-2-one;
* 5-ethoxy-1-[4-(N-*tert*-butylcarbamoyl)-2-methoxybenzenesulphonyl]-3-spiro-[4-(2-morpholinoethyloxy)-cyclohexane]indolin-2-one;
* 5-ethoxy-3-spiro-(4-carboxymethyloxycyclohexane)-1-(4-N-*tert*-butylcarbamoyl-2-methoxybenzenesulphonyl)-indolin-2-one;
* 5-ethoxy-3-spiro-[4-(2-morpholinoethyloxy)cyclohexane]-1-[4-(N-*tert*-amylcarbamoyl)-2-methoxybenzenesulphonyl]indolin-2-one;
* 5-ethoxy-3-spiro-[4-(2-carboxyethyloxy)cyclohexane]-1-[4-(N-*tert*-amylcarbamoyl)-2-methoxybenzenesulphonyl]indolin-2-one;
* 5-ethoxy-1-[4-(N',N'-diethylureido)-2-methoxybenzenesulphonyl]-3-spiro-[4-(2-dimethylaminoethyloxy)-cyclohexane]indolin-2-one;
* 5-Ethoxy-3-spiro-[4-(2-(4-ethoxypiperidino)-ethyloxy)cyclohexane]-1-[4-(N-*tert*-butylcarbamoyl)-2-methoxybenzenesulfonyl]indolin-2-one ;
* 5-Ethoxy-3-spiro-[4-(2-glycylaminoethyloxy)-cyclohexane]-1-[4-(N-*tert-*butylcarbamoyl)-2-methoxybenzenesulfonyl]indolin-2-one ;
* 5-Ethoxy-3-spiro-[4-(2-(N,N-dimethylglycylamino)-ethyloxy)cyclohexane]-1-[4-(N-*tert*-butylcarbamoyl)-2-methoxybenzenesulfonyl]indolin-2-one ;
* 5-Chloro-3-spiro-[4-(N-(3-dimethylaminopropyl)-carbamoylmethyloxy)cyclohexane]-1-[4-(N-*tert*-butylcarbamoyl)-2-methoxybenzenesulfonyl]indolin-2-one ;
* 5-Ethoxy-3-spiro-[4-(2-(4-dimethylaminobutyrylamino)ethyloxy)cyclohexane]-1-[4-(N-*tert*-butylcarbamoyl)-2-methoxybenzenesulfonyl]indolin-2-one ;
* 5-Ethoxy-3-spiro-[4-(2-(2-hydroxyethylamino)-ethyloxy)cyclohexane]-1-[4-(N-*tert*-butylcarbamoyl)-2-methoxybenzenesulfonyl]indolin-2-one ;
* 5-Ethoxy-3-spiro-[4-(2-(2-(2-hydroxyethyloxy)-ethylamino)ethyloxy)cyclohexane]-1-[4-(N-*tert*-butylcarbamoyl)-2-methoxybenzenesulfonyl]indolin-2-one; or their pharmaceutically acceptable salts, solvates or hydrates.

7. Compound according to claim 1, **characterized in that** it is the 5-ethoxy-l-[4-(N-tert-butylcarbamoyl)-2-methoxybenzenesulfonyl]-3-spiro-[4-(2-morpholinoethyloxy)-cyclohexane]indolin-2-one;
and its pharmaceutically acceptable salts, solvates or hydrates.

8. Process for the preparation of a compound of formula (I) according to any one of Claims 1 to 3, 6 and 7, **characterized in that**:
(1) either when Z = NR₁₁R₁₂, in which R₁₁ and R₁₂ are as defined for (I) :
(1a) when at least one of the R₁₁ and R₁₂ radicals is different from hydrogen, a compound of formula: 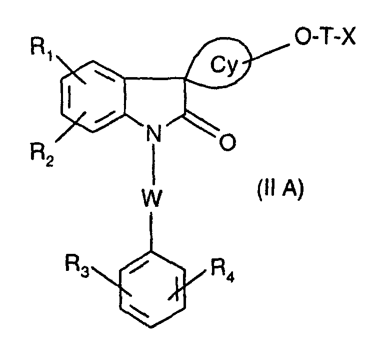 in which:
- Cy forms with the carbon atom to which it is bonded a cyclohexane substituted in 4-position with-O-T-X;
- R₂ is a hydrogen atom;
- W is SO₂;
- R₁, R₃, R₄ and T are as defined for (I.2) in claim 1; and
- X represents a halogen, a mesyloxy or a tosyloxy; is reacted with a derivative of formula ZH in a solvent selected from dimethylformamide, tetrahydrofuran or acetonitrile, at temperatures of between 0° and 120°C ;
(1b) When R₁₁ and R₁₂ = H, the compound (IIA), in which X is an azido, is reduced to amino;
(2) or, when Z = -COOH, a compound of formula: 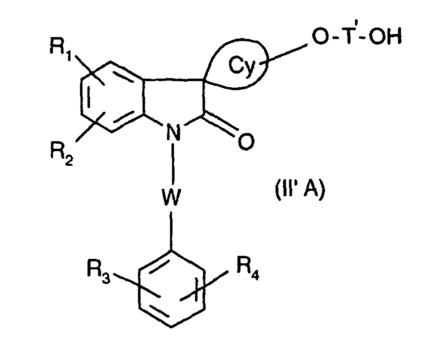 in which R₁, R₂, W, R₃, R₄ are as above defined and Cy forms with the carbon atom to which it is bonded a cyclohexane substituted in 4-position with -O-T'-OH, in which T' represents T-CH₂-, is oxidized in an acid solvent at a temperature of between 0°C and 100°C;
(3) or a compound of formula: 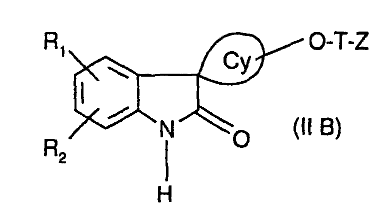 in which R₁, R₂, T and Z are as above defined and Cy forms with the carbon atom to which it is bonded a cyclohexane substituted in 4-position by -O-T-Z, is reacted with a compound of formula: 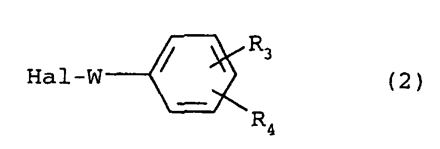 in which W, R₃ and R₄ are as above defined and Hal represents a halogen atom, in an anhydrous solvent in the presence of a metal hydride or an alkali metal alkoxide at temperatures of between -40° and 25°C;
(4) or, when Z = -COOH, a compound of formula: 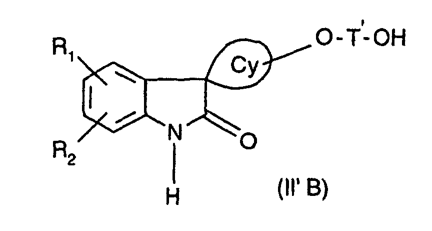 in which R₁ and R₂ are as above defined and Cy forms with the carbon atom to which it is bonded a cyclohexane substituted in 4-position by -O-T'-OH, in which T' represents T-CH₂, is oxidized, then the acid thus obtained of formula: 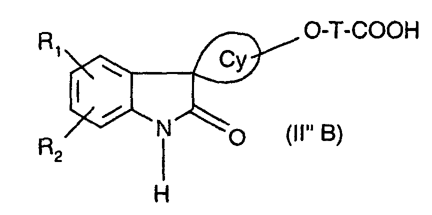 in which R₁, R₂ and T are as above defined and Cy forms with the carbon atom to which it is bonded a cyclohexane substituted in 4-position by -O-T-COOH protected by a protective group for the carboxylic acid, in order to obtain the intermediate of formula: 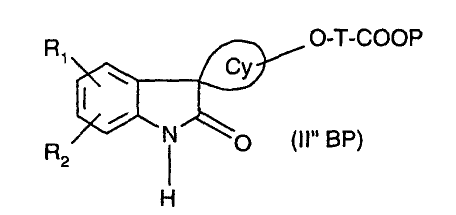 in which R₁, R₂ and T are as above defined and Cy forms with the carbon atom to which it is bonded a cyclohexane substituted in 4-position by -O-T-COOP, in which P represents a protective group chosen from an alkyl, a *tert*-butyl or a benzyl, and, finally, this compound (II"BP) is subjected to the action of a derivative of formula (2) in order to obtain, after deprotection, a compound (I.2); one of its quaternary ammoniums, oxides, sulphones or salts.

9. Pharmaceutical composition containing, as active principle, a compound of formula (I.2) according to Claim 1 or one of its pharmaceutically acceptable salts, hydrates or solvates.

10. Pharmaceutical composition containing, as active principle, a compound of formula (I.3) according to Claim 2 or one of its pharmaceutically acceptable salts, hydrates or solvates.

11. Pharmaceutical composition containing, as active principle, a compound of formula (I.4) according to Claim 3 or one of its pharmaceutically acceptable salts, hydrates or solvates.

12. Pharmaceutical composition containing, as active principle, a compound according to Claim 6.

13. Pharmaceutical composition according to any one of Claims 9 to 12 also containing another active principle.

14. Pharmaceutical composition according to Claim 13, **characterized in that** the other active principle is a specific antagonist of the angiotensin II receptor.

15. Pharmaceutical composition according to Claim 14, **characterized in that** the specific antagonist of the angiotensin II receptor is irbesartan.

16. Pharmaceutical composition containing a combination of 5-ethoxy-1-[4- (N-*tert*-butylcarbamoyl)-2-methoxybenzenesulphonyl]-3-spiro-[4-(2-morpholinoethyloxy)cyclohexane]-indolin-2-one and irbesartan.

17. Compound according to anyone of Claims 1 to 3, 6 and 7 for its use as drug.

18. Use of a compound according to anyone of claims 1 to 3, 6 and 7 for the preparation of drugs to be used for treating vasopressine-dependent or ocytocine-dependent complaints.

19. Use of a compound according to anyone of claims 1 to 3, 6 and 7 for the preparation of drugs to be used for treating cardiac insufficiency.

20. Use of a compound according to anyone of claims 1 to 3, 6 and 7 for the preparation of drugs to be used for treating glaucoma.

21. Use of a composition according to anyone of Claims 9 to 16 for the preparation of drugs to be used for treating or preventing cardiac insufficiency.

22. Use of a composition according to anyone of Claims 9 to 16 for the preparation of drugs to be used for treating or preventing the syndrome of inappropriate secretion of antidiuretic hormone (SIADH).

## Patentansprüche

1. Verbindung der Formel 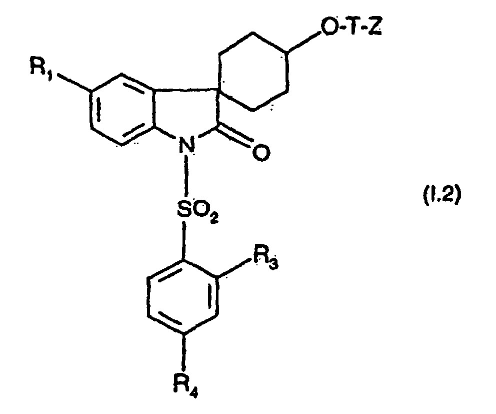 in der:
- R₁ Wasserstoff; Hydroxyl; Halogen; (C₁-C₇)-Alkyl; (C₁-C₇)-Polyfluoralkyl; (C₁-C₇)-Alkoxy; (C₁-C₇) -Alkylthio; (C₁-C₇)-Polyfluoralkoxy; (C₃-C₇)-Cycloalkyloxy; (C₃-C₇)-Cycloalkylthio; Cycloalkylmethoxy oder Cycloalkylmethylthio darstellt, in denen das Cycloalkyl C₃-C₇; Phenoxy; Benzyloxy; Nitro; Cyano ist;
- R₃ und R₄ stellen jeweils unabhängig voneinander Wasserstoff; Halogen; (C₁-C₇) -Alkyl; (C₂-C₇)-Alkenyl; (C₁-C₇)-Polyhalogenalkyl; Phenyl oder Benzyl; Cyano; Nitro; eine -NR₅R₆-Gruppe; Hydroxyamino; Hydroxyl; eine OR₇-Gruppe; eine SR₇-Gruppe; eine -COOR₈-Gruppe; eine -CONR₉R₁₀-Gruppe; eine -CSNR₉R₁₀-Gruppe dar; wobei mindestens eines der R₃- und R₄-Radikale verschieden von Wasserstoff ist;
- R₅ und R₆ stellen jeweils unabhängig voneinander Wasserstoff; (C₁-C₇)-Alkyl; (C₂-C₇)-Alkenyl; Phenyl; Benzyl; (C₁-C₇)-Alkylcarbonyl; (C₁-C₇)-Alkylthiocarbonyl; (C₃-C₇) -Cycloalkylcarbonyl; (C₃-C₇)-Cycloalkylthiocarbonyl; Benzoyl; Thienylcarbonyl; Furylcarbonyl; (C₁-C₇)-Alkyloxycarbonyl; Phenoxycarbonyl; Benzyloxycarbonyl; Carbamoyl oder Thiocarbamoyl, die nicht substituiert oder substituiert sind mit R₉ und R₁₀ dar; oder aber R₅ und R₆ bilden mit dem Stickstoffatom, an das sie gebunden sind, eine heterozyklische Gruppe, die ausgewählt ist aus den Gruppen Pyrrolidin, Pyrrolin, Pyrrolyl, Indolin, Indol, Piperidin;
- R₇ stellt ein (C₁-C₇)-Alkyl; (C₂-C₇)-Alkenyl; Phenyl; Benzyl; (C₃-C₇) -Cycloalkyl; (C₁-C₇) -Polyfluoralkyl; Formyl; (C₁-C₇)-Alkylcarbonyl; Benzoyl; Benzylcarbonyl dar;
- R₈ stellt Wasserstoff, (C₁-C₇)-Alkyl; Phenyl; Benzyl dar;
- R₉ und R₁₀ stellen jeweils unabhängig voneinander Wasserstoff; (C₁-C₇)-Alkyl; (C₁-C₇)-Polyfluoralkyl; (C₂-C₇)-Alkenyl; (C₃-C₇)-Cycloalkyl, das gegebenenfalls mit einer Hydroxy-(C₁-C₄)-Alkylgruppe substituiert ist; Pyridyl; Phenyl; Thienyl; Furyl; (C₄-C₇)-Azacycloalkyl dar, oder R₉ und R₁₀ bilden mit dem Stickstoffatom, mit dem sie verbunden sind, eine heterozyklische Gruppe, die ausgewählt aus den Gruppen Pyrrolidin, Piperidin und Piperazin, nicht-substituiert oder substituiert mit (C₁-C₄) -Alkylen;
- T stellt (C₁-C₄)-Alkylen dar, gegebenenfalls unterbrochen von einem (C₃-C₆)-Cycloalkylen, wobei die Alkylene gegebenenfalls einmal oder mehrmals am gleichen Kohlenstoffatom mit einem (C₁-C₃)-Alkyl substituiert sind; oder T stellt eine direkte Bindung dar;
- Z stellt eine -NR₁₁R₁₂-Gruppe dar; -⁺NR₁₁R₁₂-(C₁-C₄) - Alkyl-(A⁻), (A⁻) ist ein Anion; -N(O)R₁₁R₁₂; eine -COOR₁₁-Gruppe; eine -NR₁₁COR₁₂-Gruppe; eine -CONR₁₁R₁₂-Gruppe, vorausgesetzt, dass, wenn T ein Methylen oder eine direkte Bindung darstellt, Z nicht -NR₁₁R₁₂; -⁺(NR₁₁R₁₂(C₁-C₄)-Alkyl (A⁻) ; -N(O)R₁₁R₁₂; NR₁₁COR₁₂ sein kann;
- R₁₁ und R₁₂ stellen jeweils unabhängig voneinander Wasserstoff; (C₁-C₇)-Alkyl; (C₁-C₄)-Alkoxy; (C₃-C₇)-Cycloalkyl; Phenyl; (C₁-C₃)-Alkylencycloalkyl dar, in denen das Cycloalkyl C₃-C₇ ist; (C₁-C₃)-Alkylenphenyl; wobei die Gruppen gegebenenfalls mit R₁₃ mono- oder polysubstituiert sein können;
oder R₁₁ und R₁₂ bilden gegebenenfalls mit dem Stickstoffatom, an das sie gebunden sind, einen Heterozyklus, der ausgewählt wird aus den Heterozyklen Azetidin, Pyrrolidin, Piperidin, Piperazin, Piperazinon, Morpholin, Morpholinon, Thiomorpholin, Hexahydroazepin, gegebenenfalls monosubstituiert mit R₁₃;
- R₁₃ stellt dar (C₁-C₄)-Alkyl; Wasserstoff; Hydroxyalkyloxy; (C₁-C₄)-Alkoxy; eine -NR₁₄R₁₅-Gruppe, in denen R₁₄ und R₁₅ jeweils unabhängig voneinander Wasserstoff oder ein (C₁-C₄)-Alkyl; Amidino; Guanidino; Imidazolyl; Pyridyl; Indolyl; Tetrahydroisochinolyl darstellen;
- die Phenylgruppe, die Substituenten R₁, R₃, R₄, R₅, R₆, R₇, R₈, R₉, R₁₀, R₁₁ und R₁₂ bildet, ist nicht substituiert, mono- oder disubstituiert mit einem (C₁-C₇)-Alkyl, einem (C₁-C₇)-Alkoxy, Trifluormethyl, einem Halogen, oder trisubstituiert mit einem (C₁-C₇)-Alkyl, einem (C₁-C₇)-Alkoxy oder einem Halogen;

2. Verbindung nach Anspruch 1 mit der Formel: 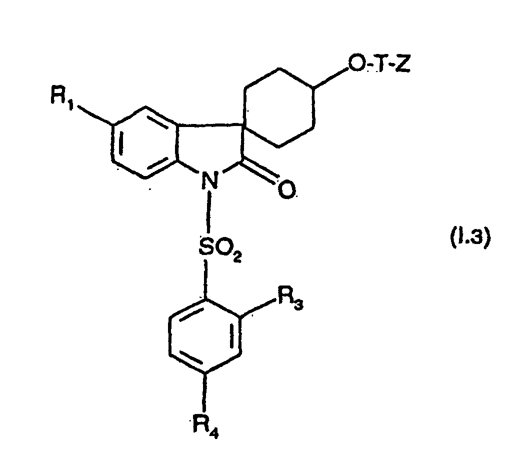 in der R₁, R₃ und R₄ so sind, wie für (I.2) im Anspruch 1 definiert, T stellt ein (C₁-C₄)-Alkylen dar und Z stellt eine Aminogruppe, ein 2-Hydroxyethylamino, ein 2-(2-Hydroxy)ethyloxyethylamino, ein Morpholinyl oder ein Carboxy, und seine Salze, Solvate oder Hydrate dar.

3. Verbindung nach Anspruch 1 mit der Formel: 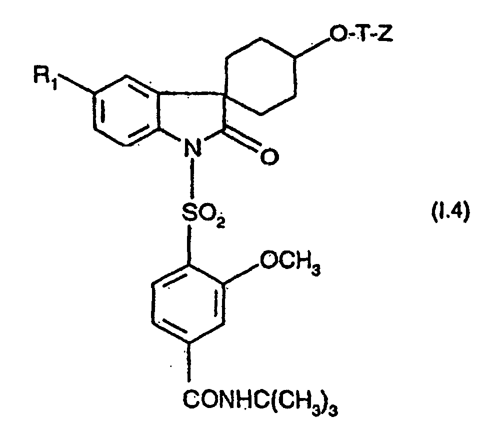 in der R₁, T und Z so sind, wie im Anspruch 1 für (I.2) definiert, oder eines seiner Salze, Solvate oder Hydrate.

4. Verbindung der Formel: 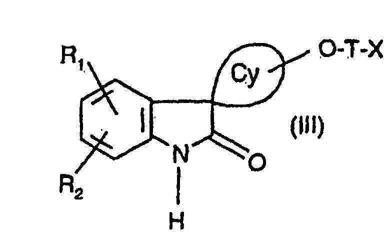 in der:
- Cy mit dem Kohlenstoff, mit dem es verbunden ist, ein Cyclohexan bildet, das an der Position 4 mit -O-T-X substituiert ist;
- R₂ ist ein Wasserstoffatom;
- R₁ und T sind wie im Anspruch 1 für (I.2) definiert; und
- X ist Halogen, Mesyloxy oder Tosyloxy; oder aber X stellt eine Azidogruppe, oder eines seiner Salze, Solvate oder Hydrate dar.

5. Verbindung gemäß Anspruch 4, **dadurch gekennzeichnet, dass** sie eine der folgenden Verbindungen ist:
* 5-Ethoxy-3-spiro-[4-(3-chlorpropyloxy)cyclohexan]indolin-2-on;
* 5-Ethoxy-3-spiro-[(4-(2-chlorethyloxy)cyclohexan]indolin-2-on;
* 5-Chlor-3-spiro-[4-(2-chlorethyloxy)cyclohexan]inolin-2-on;
* 5-Ethoxy-3-spiro-[4-(2-tosyloxyethyloxy)cyclohexan]indolin-2-on.

6. Verbindung gemäß Anspruch 1, **dadurch gekennzeichnet, dass** sie eine der nachfolgenden Verbindungen ist:
* 5-Chlor-3-spiro-[4-(2-morpholinoethyloxy)cyclohexan]-1-[4-(N-tert-butylcarbamoyl)-2-methoxybenzolsulfonyl]indolin-2-on;
* 5-Ethoxy-3-spiro-[4-(2-aminoethyloxy)cyclohexan]-1-[4-(4-N-tert-butylcarbamoyl)-2-methoxybenzolsulfonyl]indolin-2-on;
* 5-Ethoxy-3-spiro-[4-(2-(N-methyl-N-(2-hydroxyethyl)amino)ethyloxy)cyclohexan]-1-[4-(Ntert-butylcarbamoyl)-2-methoxybenzolsulfonyl]indolin-2-on;
* 5-Ethoxy-1-[4-(N-tert-butylcarbamoyl))-2-methoxybenzolsulfonyl]-3-spiro-[4-(2-morpholinoethyloxy)cyclohexan]indolin-2-on;
* 5-Ethoxy-3-spiro-(4-carboxymethyloxycyclohexan)-1-(4-N-tert-butylcarbamoyl-2-methoxybenzolsulfonyl)indolin-2-on;
* 5-Ethoxy-3-spiro[4-(2-morpholinoethyloxy)cyclohexan]-1-[4-(N-tert-amylcarbamoyl)-2-methoxybenzolsulfonyl]indolin-2-on;
* 5-Ethoxy-4-spiro-[4-(2-carboxyethyloxy)cyclohexan]-1-[4-(N-tert-amylcarbamoyl)-2-methoxybenzolsulfonyl]indolin-2-on;
* 5-Ethoxy-1-[4-(N',N'-diethylureido)-2-methoxybenzolsulfonyl]-3-spiro-[4-(2-dimethylaminoethyloxy)cyclohexan]indolin-2-on;
* 5-Ethoxy-3-spiro-[4-(2-(4-ethoxypiperidino)ethyloxy)cyclohexan]-1-[4-(N-tertbutylcarbamoyl)-2-methoxybenzolsulfonyl]indolin-2-on;
* 5-Ethoxy-3-spiro-[4-(2-glycylaminoethyloxy)cyclohexan]-1-(4-(N-tertbutylcarbamoyl)-2-methoxybenzolsulfonyl]indolin-2-on;
* 5-Ethoxy-3-spiro-[4-(2-(N,N-dimethylglycylamino)ethyloxy)cyclohexan]-1-[4-(Ntert-butylcarbamoyl)-2-methoxybenzolsulfonyl]indolin-2-on;
* 5-Chlor-3-spiro-[4-(N-(3-dimethylaminopropyl)carbamoylmethyloxy)cyclohexan]-1-[4-(N-tert-butylcarbamoyl)-2-methoxybenzolsulfonyl]indolin-2-on;
* 5-Ethoxy-3-spiro-[4-(2-(4-dimethylaminobutyrylamino)ethyloxy)cyclohexan]-1-[4-(N-tert-butylcarbamoyl)-2-methoxybenzolsulfonyl]indolin-2-on;
* 5-Ethoxy-3-spiro-[4-(2-(2-hydroxyethylamino)ethyloxy)cyclohexan]-1-[4-(Ntert-butylcarbamoyl)-2-methoxybenzolsulfonyl]indolin-2-on;
* 5-Ethoxy-3-spiro-[4-(2-(2-(2-hydroxyethyloxy)ethylamino)ethyloxy)cyclohexan]-1-[4-(N-tert-butylcarbamoyl)-2-methoxybenzolsulfonyl]indolin-2-on; oder deren Salze, Solvate oder pharmazeutisch akzeptable Hydrate.

7. Verbindung gemäß Anspruch 1, **dadurch gekennzeichnet, dass** es 5-Ethoxy-1-[4-(N-tert-butylcarbamoyl)-2-methoxybenzolsulfonyl]-3-spiro-[4-(2-morpholinoethyloxy)cyclohexan]indolin-2-on ist, sowie dessen Salze, Solvate oder pharmazeutisch akzeptable Hydrate.

8. Verfahren zur Herstellung einer Verbindung gemäß einem der Ansprüche 1 bis 3, 6 und 7, **dadurch gekennzeichnet, dass**:
(1) wenn Z entweder = NR₁₁R₁₂, in denen R₁₁ und R₁₂ wie für (I.2) definiert sind:
(1a) wenn mindestens eins der Radikale R₁₁ und R₁₂ unterschiedlich vom Wasserstoff ist, lässt man miteiner Verbindung der Formel 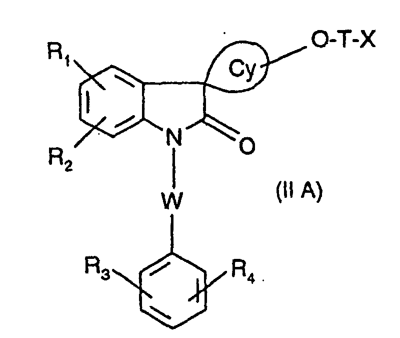 reagieren, in denen:
- Cy mit dem Kohlenstoff, mit dem es verbunden ist, ein Cyclohexan bildet, das an der Position 4 mit -O-T-X substituiert ist;
- R₂ ist ein Wasserstoffatom;
- W ist SO₂;
- R₁, R₃, R₄ und T sind wie im Anspruch 1 für (I.2) definiert; und
- X stellt ein Halogen, ein Mesyloxy oder ein Tosyloxy dar, mit einem Derivat der Formel ZH, in einem Lösungsmittel, das ausgewählt wird aus Dimethylformamid, Tetrahydrofuran oder Acetonitril, bei Temperaturen zwischen 0 und 120°C;
(1b) wenn R₁₁ und R₁₂ = H, ist die Verbindung (IIA), in der X ein Azido ist, am Amino reduziert;
(2) oder wenn Z = -COOH ist, oxidiert man eine Verbindung der Formel: 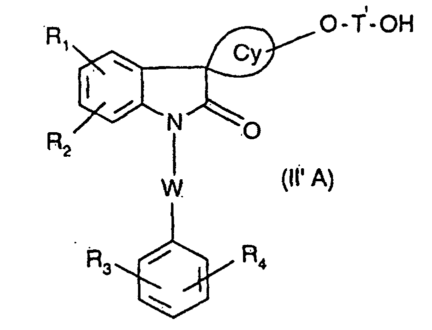 in der R₁, R₂, W, R₃, R₄ wie oben definiert sind, und Cy mit dem Kohlenstoff, mit dem es verbunden ist, ein Cyclohexan bildet, das an der Position 4 mit O-T'-OH substituiert ist, indem T' T-CH₂- in einem sauren Lösungsmittel bei einer Temperatur zwischen 0°C und 100°C darstellt;
(3) oder man setzt eine Verbindung der Formel 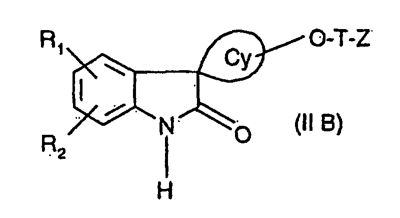 um, in der R₁, R₂, T und Z wie oben definiert sind und Cy mit dem Kohlenstoffatom, mit dem es verbunden ist, ein Cyclohexan bildet, das an der Position 4 mit -O-T-Z substituiert ist, mit einer Verbindung der Formel: 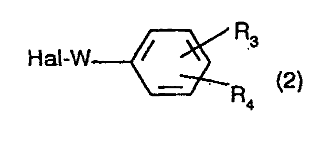 in der W, R₃ und R₄ wie oben definiert sind, und Hal ein Halogenatom in einem wasserfreien Lösungsmittel in Gegenwart eines Metallhydrats oder eines alkalischen Alkoholats bei Temperaturen zwischen -40°C und 25°C darstellt;
(4) oder wenn Z = -COOH, oxidiert man eine Verbindung der Formel: 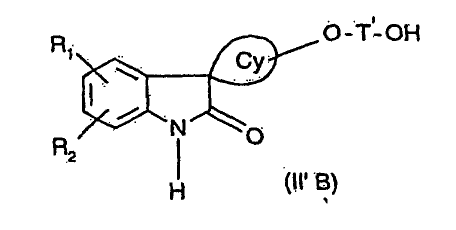 in der R₁ und R₂ wie oben definiert sind, und Cy mit dem Kohlenstoffatom, mit dem es verbunden ist, ein Cyclohexan bildet, das an der Position 4 mit -O-T'-OH substituiert ist, in dem T' T-CH₂ darstellt, dann schützt man anschließend gegebenenfalls die so erhaltene Säure mit der Formel: 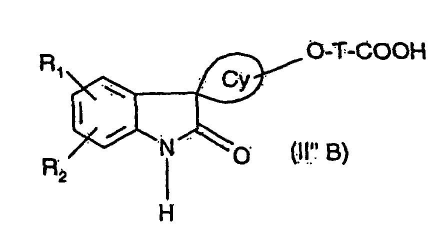 in der R₁, R₂ und T wie oben definiert sind, und Cy mit dem Kohlenstoffatom, mit dem es verbunden ist, ein Cyclohexan bildet, das an der Position 4 mit -O-T-COOH substituiert ist, worin die -O-T-COOH Gruppe mit einer Schutzgruppe der Carbonsäure geschützt ist, um das Zwischenprodukt der Formel zu erhalten: 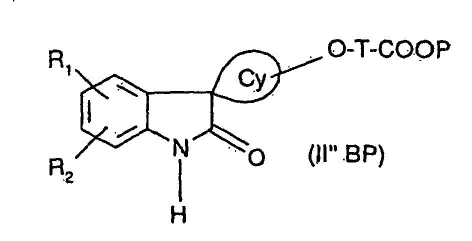 in der R₂, R₂ und T wie oben definiert sind, Cy mit dem Kohlenstoffatom, mit dem es verbunden ist, ein Cyclohexan bildet, das an der Position 4 mit -O-T-COOP substituiert ist, in dem P eine Schutzgruppe darstellt, die ausgewählt ist aus einem Alkyl, einem tert-Butyl, oder einem Benzyl und man unterwirft diese Verbindung (II"BP) dann der Einwirkung eines Derivats der Formel (2), um nach der Entschützung eine Verbindung (I.2) zu erhalten, eines derer quaternären Oxide, Sulfone oder Salze.

9. Pharmazeutische Zusammensetzung, die als aktiven Wirkstoff eine Verbindung der Formel (I.2) nach Anspruch 1 oder eines pharmazeutisch akzeptablen der Salze, Hydrate oder Solvate, enthält.

10. Pharmazeutische Zusammensetzung, die als aktiven Wirkstoff eine Verbindung der Formel (I.3) nach Anspruch 2 oder eines der pharmazeutisch akzeptablen Salze, Hydrate oder Solvate enthält.

11. Pharmazeutische Zusammensetzung, die als aktiven Wirkstoff eine Verbindung der Formel (I.4) gemäß Anspruch 3 oder eines der pharmazeutisch akzeptablen Salze, Hydrate oder Solvate enthält.

12. Pharmazeutische Verbindung, die als aktiven Wirkstoff eine Verbindung nach Anspruch 6 enthält.

13. Pharmazeutische Zusammensetzung gemäß einem der Ansprüche 9 bis 12, die außerdem einen weiteren aktiven Wirkstoff enthält.

14. Pharmazeutische Zusammensetzung gemäß Anspruch 13, **dadurch gekennzeichnet, dass** der andere aktive Wirkstoff ein spezifischer Antagonist des Angiotensin II-Rezeptors ist.

15. Pharmazeutische Zusammensetzung gemäß Anspruch 14, **dadurch gekennzeichnet, dass** der spezifische Antagonist des Angiotensin II-Rezeptors Irbesartan ist.

16. Pharmazeutische Zusammensetzung, die eine Verbindung aus 5-Ethoxy-1-[4-(N-tert-butylcarbamoyl))-2-methoxybenzolsulfonyl]-3-spiro-[4-(2-morpholinoethyloxy)cyclohexan]indolin-2-on und Irbesartan enthält.

17. Verbindung gemäß einem der Ansprüche 1 bis 3, 6 und 7 für dessen Verwendung als Medikament.

18. Verwendung einer Verbindung gemäß einem der Ansprüche 1 bis 3, 6 und 7 zur Herstellung von Medikamenten, die darauf ausgerichtet sind, Vasopressin-abhängige oder Oxytoxin-abhängige Erkrankungen zu behandeln.

19. Verwendung einer Verbindung gemäß einem der Ansprüche 1 bis 3, 6 und 7 zur Herstellung von Medikamenten, die darauf ausgerichtet sind, die Herzinsuffizienz zu behandeln.

20. Verwendung einer Verbindung gemäß einem der Ansprüche 1 bis 3, 6 und 7 zur Herstellung von Medikamenten, die darauf ausgerichtet sind, ein Glaukom zu behandeln.

21. Verwendung einer Verbindung gemäß einem der Ansprüche 9 bis 16 zur Herstellung von Medikamenten, die darauf ausgerichtet sind, die Herzinsuffizienz zu behandeln oder zu verhindern.

22. Verwendung einer Zusammensetzung gemäß einem der Ansprüche 9 bis 16 zur Herstellung von Medikamenten, die darauf ausgerichtet sind, das Syndrom der inappropriaten ADH-Sekretion (SIADH) zu behandeln oder zu verhindern.
